# EUROPEAN PATENT SPECIFICATION

(11) **EP 1 753 427 B1**
(45) Date of publication and mention of the grant of the patent: **02.04.2008**
(21) Application number: 05743054.8
(22) Date of filing: 24.05.2005
(51) Int. Cl.: A61K 31/47, A61P 31/06

(54) **USE OF SUBSTITUTED QUINOLINE DERIVATIVES FOR THE TREATMENT OF DRUG RESISTANT MYCOBACTERIAL DISEASES**
VERWENDUNG VON SUBSTITUIERTEN CHINOLIN-DERIVATEN ZUR BEHANDLUNG VON ARZNEIMITTELRESISTENTEN MYCOBAKTERIELLEN ERKRANKUNGEN
UTILISATION DE DERIVES SUBSTITUES DE QUINOLINE DANS LE TRAITEMENT DE MALADIES MYCOBACTERIENNES RESISTANTES AUX MEDICAMENTS

(30) Priority: 28.05.2004 EP 04102402
(43) Date of publication of application: 21.02.2007
(73) Proprietor: Janssen Pharmaceutica NV, 2340 Beerse (BE)
(72) Inventor: ANDRIES, Koenraad, Jozef, Lodewijk, Marcel, B-2340 Beerse (BE); VAN GESTEL, Jozef, Frans, Elisabetha, B-2350 Vosselaar (BE)
(74) Representative: Vervoort, Liesbeth
(86) International application number: PCT/EP2005/052371
(87) International publication number: WO 2005/117875

(56) References cited:
- WO-A-20/04011436

## Description

The present invention relates to the use of substituted quinoline derivatives for inhibiting the growth of drug resistant Mycobacterium strains including growth inhibition of multi drug resistant Mycobacterium strains. The substituted quinoline derivatives can thus be used for the treatment or the prevention of Mycobacterial diseases caused by drug resistant, particularly multi drug resistant Mycobacteria. More in particular the present quinoline derivatives can be used for the treatment or the prevention of Mycobacterial diseases caused by drug resistant including multi drug resistant *Mycobacterium tuberculosis.* The present invention also relates to a combination of (a) a substituted quinoline derivative according to the present invention and (b) one or more other antimycobacterial agents.

### BACKGROUND OF THE INVENTION

*Mycobacterium tuberculosis* is the causative agent of tuberculosis (TB), a serious and potentially fatal infection with a world-wide distribution. Estimates from the World Health Organization indicate that more than 8 million people contract TB each year, and 2 million people die from tuberculosis yearly. In the last decade, TB cases have grown 20% worldwide with the highest burden in the most impoverished communities. If these trends continue, TB incidence will increase by 41% in the next twenty years. Fifty years since the introduction of an effective chemotherapy, TB remains after AIDS, the leading infectious cause of adult mortality in the world. Complicating the TB epidemic is the rising tide of multi-drug- resistant strains, and the deadly symbiosis with HIV. People who are HIV-positive and infected with TB are 30 times more likely to develop active TB than people who are HIV-negative and TB is responsible for the death of one out of every three people with HIV/AIDS worldwide

Existing approaches to treatment of tuberculosis all involve the combination of multiple agents. For example, the regimen recommended by the U.S. Public Health Service is a combination of isoniazid, rifampicin and pyrazinamide for two months, followed by isoniazid and rifampicin alone for a further four months. These drugs are continued for a further seven months in patients infected with HIV. For patients infected with multi-drug resistant strains of *M*. *tuberculosis,* agents such as ethambutol, streptomycin, kanamycin, amikacin, capreomycin, ethionamide, cycloserine, ciprofoxacin and ofloxacin are added to the combination therapies. There exists no single agent that is effective in the clinical treatment of tuberculosis, nor any combination of agents that offers the possibility of therapy of less than six months' duration.

There is a high medical need for new drugs that improve current treatment by enabling regimens that facilitate patient and provider compliance. Shorter regimens and those that require less supervision are the best way to achieve this. Most of the benefit from treatment comes in the first 2 months, during the intensive, or bactericidal, phase when four drugs are given together; the bacterial burden is greatly reduced, and patients become noninfectious. The 4- to 6-month continuation, or sterilizing, phase is required to eliminate persisting bacilli and to minimize the risk of relapse. A potent sterilizing drug that shortens treatment to 2 months or less would be extremely beneficial. Drugs that facilitate compliance by requiring less intensive supervision also are needed. Obviously, a compound that reduces both the total length of treatment and the frequency of drug administration would provide the greatest benefit

Complicating the TB epidemic is the increasing incidence of multi drug- resistant strains or MDR-TB. Up to four percent of all cases worldwide are considered MDR-TB - those resistant to the most effective drugs of the four-drug standard, isoniazid and rifampin. MDR- TB is lethal when untreated and can not be adequately treated through the standard therapy, so treatment requires up to 2 years of "second-line" drugs. These drugs are often toxic, expensive and marginally effective. In the absence of an effective therapy, infectious MDR-TB patients continue to spread the disease, producing new infections with MDR-TB strains. There is a high medical need for drugs which demonstrate activity against resistant and/or MDR strains.

The term "drug resistant" as used hereinbefore or hereinafter is a term well understood by the person skilled in microbiology. A drug resistant Mycobacterium is a Mycobacterium which is no longer susceptible to at least one previously effective drug; which has developed the ability to withstand antibiotic attack by at least one previously effective drug. A drug resistant strain may relay that ability to withstand to its progeny. Said resistance may be due to random genetic mutations in the bacterial cell that alters its sensitivity to a single drug or to different drugs.
MDR tuberculosis is a specific form of drug resistant tuberculosis due to a bacterium resistant to at least isoniazid and rifampicin (with or without resistance to other drugs), which are at present the two most powerful anti-TB drugs. Thus, whenever used hereinbefore or hereinafter "drug resistant" includes multi drug resistant.

Unexpectedly, it has now been found that the substituted quinoline derivatives of the present invention are very useful for inhibiting growth of drug resistant, in particular multi drug resistant, Mycobacteria and therefore useful for the treatment of diseases caused by drug resistant, in particular multi drug resistant, Mycobacteria, particularly those diseases caused by drug resistant, in particular multi drug resistant, pathogenic *Mycobacterium (M.) tuberculosis, M. bovis, M. avium, M. fortuitum, M. leprae and M. marinum,* more particularly *Mycobacterium tuberculosis.*

The substituted quinoline derivatives relating to the present invention were already disclosed in WO 2004/011436. Said document discloses the antimycobacterial property of the substituted quinoline derivatives against sensitive, susceptible Mycobacterium strains but is silent on their activity against drug resistant, in particular multi drug resistant, Mycobacteria.

Thus, the present invention relates to the use of a substituted quinoline derivative for the preparation of a medicament for the treatment of a warm-blooded mammal infected with a drug resistant Mycobacterium strain wherein the substituted quinoline derivative is a compound according to Formula (Ia) or Formula (Ib) 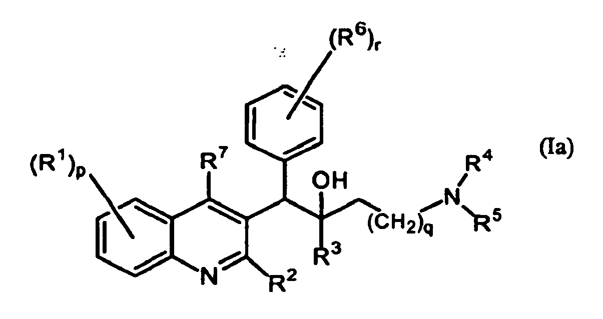 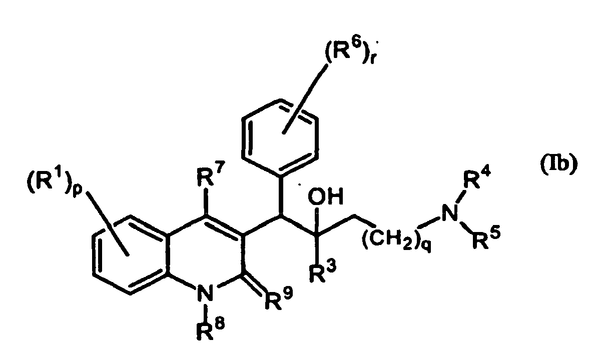 a pharmaceutically acceptable acid or base addition salt thereof, a stereochemically isomeric form thereof, a tautomeric form thereof or a *N*-oxide form thereof, wherein :
- R¹: is hydrogen, halo, haloalkyl, cyano, hydroxy, Ar, Het, alkyl, alkyloxy, alkylthio, alkyloxyalkyl, alkylthioalkyl, Ar-alkyl or di(Ar)alkyl ;
- p: is an integer equal to 1, 2, 3 or 4 ;
- R²: is hydrogen, hydroxy, mercapto, alkyloxy, alkyloxyalkyloxy, alkylthio, mono or di(alkyl)amino or a radical of formula 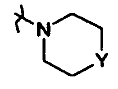 wherein Y is CH₂, O, S, NH or N-alkyl ;
- R³: is alkyl, Ar, Ar-alkyl, Het or Het-alkyl;
- q: is an integer equal to zero, 1, 2, 3 or 4 ;
- R⁴ and R⁵: each independently are hydrogen, alkyl or benzyl; or
- R⁴ and R⁵: together and including the N to which they are attached may form a radical selected from the group of pyrrolidinyl, 2*H*-pyrrolyl, 2-pyrrolinyl, 3-pyrrolinyl, pyrrolyl, imidazolidinyl, pyrazolidinyl, 2-imidazolinyl, 2-pyrazolinyl, imidazolyl, pyrazolyl, triazolyl, piperidinyl, pyridinyl, piperazinyl, imidazolidinyl, pyridazinyl, pyrimidinyl, pyrazinyl, triazinyl, morpholinyl and thiomorpholinyl, optionally substituted with alkyl, halo, haloalkyl, hydroxy, alkyloxy, amino, mono- or dialkylamino, alkylthio, alkyloxyalkyl, alkylthioalkyl and pyrimidinyl ;
- R⁶: is hydrogen, halo, haloalkyl, hydroxy, Ar, alkyl, alkyloxy, alkylthio, alkyloxyalkyl, alkylthioalkyl, Ar-alkyl or di(Ar)alkyl ; or
- two vicinal R⁶: radicals may be taken together to form a bivalent radical of formula -CH=CH-CH=CH- ;
- r: is an integer equal to 1, 2, 3, 4 or 5 ; and
- R⁷: is hydrogen, alkyl, Ar or Het;
- R⁸: is hydrogen or alkyl ;
- R⁹: is oxo ; or
- R⁸ and R⁹: together form the radical =N-CH=CH-;
- alkyl: is a straight or branched saturated hydrocarbon radical having from 1 to 6 carbon atoms ; or is a cyclic saturated hydrocarbon radical having from 3 to 6 carbon atoms ; or is a a cyclic saturated hydrocarbon radical having from 3 to 6 carbon atoms attached to a straight or branched saturated hydrocarbon radical having from 1 to 6 carbon atoms ; wherein each carbon atom can be optionally substituted with halo, hydroxy, alkyloxy or oxo ;
- Ar: is a homocycle selected from the group of phenyl, naphthyl, acenaphthyl, tetrahydronaphthyl, each optionally substituted with 1,2 or 3 substituents, each substituent independently selected from the group of hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl and mono- or dialkylaminocarbonyl ;
- Het: is a monocyclic heterocycle selected from the group of *N*-phenoxypiperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl and pyridazinyl; or a bicyclic heterocycle selected from the group of quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl or benzo[1,3]dioxolyl ; each monocyclic and bicyclic heterocycle may optionally be substituted on a carbon atom with 1, 2 or 3 substituents selected from the group of halo, hydroxy, alkyl or alkyloxy ;
- halo: is a substituent selected from the group of fluoro, chloro, bromo and iodo and
- haloalkyl: is a straight or branched saturated hydrocarbon radical having from 1 to 6 carbon atoms or a cyclic saturated hydrocarbon radical having from 3 to 6 carbon atoms, wherein one or more carbon atoms are substituted with one or more halo-atoms.

More in particular, the present invention relates to the use of a substituted quinoline derivative for the preparation of a medicament for the treatment of an infection with a drug resistant Mycobacterium strain wherein the substituted quinoline derivative is a compound according to Formula (Ia) or Formula (Ib).

The compounds according to Formula (Ia) and (Ib) are interrelated in that e.g. a compound according to Formula (Ib), with R⁹ equal to oxo is the tautomeric equivalent of a compound according to Formula (Ia) with R² equal to hydroxy (keto-enol tautomerism).

In the framework of this application, alkyl is a straight or branched saturated hydrocarbon radical having from 1 to 6 carbon atoms ; or is a cyclic saturated hydrocarbon radical having from 3 to 6 carbon atoms ; or is a cyclic saturated hydrocarbon radical having from 3 to 6 carbon atoms attached to a straight or branched saturated hydrocarbon radical having from 1 to 6 carbon atoms ; wherein each carbon atom can be optionally substituted with halo, hydroxy, alkyloxy or oxo.
Preferably, alkyl is methyl, ethyl or cyclohexylmethyl.

In the framework of this application, Ar is a homocycle selected from the group of phenyl, naphthyl, acenaphthyl, tetrahydronaphthyl, each optionally substituted with 1, 2 or 3 substituents, each substituent independently selected from the group of hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl and mono- or dialkylaminocarbonyl. Preferably, Ar is naphthyl or phenyl, each optionally substituted with 1 or 2 halo substituents.

In the framework of this application, Het is a monocyclic heterocycle selected from the group of *N*-phenoxypiperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl and pyridazinyl; or a bicyclic heterocycle selected from the group of quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl or benzo[1,3]dioxolyl ; each monocyclic and bicyclic heterocycle may optionally be substituted on a carbon atom with 1, 2 or 3 substituents selected from the group of halo, hydroxy, alkyl or alkyloxy. Preferably, Het is thienyl.

In the framework of this application, halo is a substituent selected from the group of fluoro, chloro, bromo and iodo and haloalkyl is a straight or branched saturated hydrocarbon radical having from 1 to 6 carbon atoms or a cyclic saturated hydrocarbon radical having from 3 to 6 carbon atoms, wherein one or more carbon atoms are substituted with one or more halo-atoms. Preferably, halo is bromo, fluoro or chloro and preferably, haloalkyl is trifluoromethyl. When alkyl is substituted with more than one halo atom, each halo atom may be the same or different

Preferably, the invention relates to the use as defined hereinabove of compounds of Formula (Ia) or (Ib) 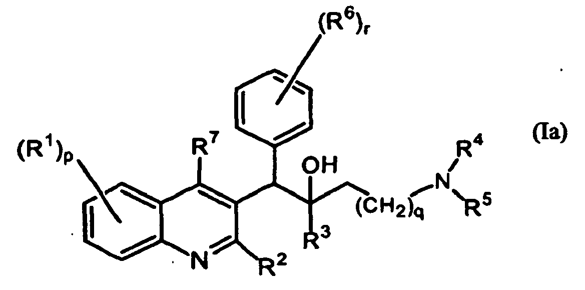 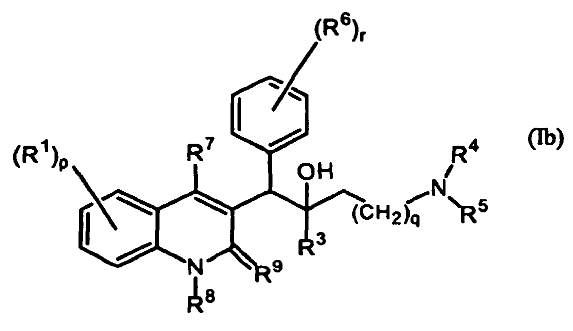 a pharmaceutically acceptable acid or base addition salt thereof, a stereochemically isomeric form thereof, a tautomeric form thereof or a *N*-oxide form thereof, wherein :
- R¹: is hydrogen, halo, haloalkyl, cyano, hydroxy, Ar, Het, alkyl, alkyloxy, alkylthio, alkyloxyalkyl, alkylthioalkyl, Ar-alkyl or di(Ar)alkyl ;
- p: is an integer equal to 1, 2, 3 or 4 ;
- R²: is hydrogen, hydroxy, mercapto, alkyloxy, alkyloxyalkyloxy, alkylthio, mono or di(alkyl)amino or a radical of formula 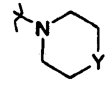 wherein Y is CH₂, O, S, NH or N-alkyl ;
- R³: is alkyl, Ar, Ar-alkyl, Het or Het-alkyl;
- q: is an integer equal to zero, 1, 2, 3 or 4 ;
- R⁴ and R⁵: each independently are hydrogen, alkyl or benzyl; or
- R⁴ and R⁵: together and including the N to which they are attached may form a radical selected from the group of pyrrolidinyl, 2*H*-pyrrolyl, 2-pyrrolinyl, 3-pyrrolinyl, pyrrolyl, imidazolidinyl, pyrazolidinyl, 2-imidazolinyl, 2-pyrazolinyl, imidazolyl, pyrazolyl, triazolyl, piperidinyl, pyridinyl, piperazinyl, imidazolidinyl, pyridazinyl, pyrimidinyl, pyrazinyl, triazinyl, morpholinyl and thiomorpholinyl, optionally substituted with alkyl, halo, haloalkyl, hydroxy, alkyloxy, amino, mono- or dialkylamino, alkylthio, alkyloxyalkyl, alkylthioalkyl and pyrimidinyl ;
- R⁶: is hydrogen, halo, haloalkyl, hydroxy, Ar, alkyl, alkyloxy, alkylthio, alkyloxyalkyl, alkylthioalkyl, Ar-alkyl or di(Ar)alkyl ; or
- two vicinal R⁶: radicals may be taken together to form a bivalent radical of formula =C-C=C=C- ;
- r: is an integer equal to 1, 2, 3, 4 or 5 ; and
- R⁷: is hydrogen, alkyl, Ar or Het ;
- R⁸: is hydrogen or alkyl ;
- R⁹: is oxo ; or
- R⁸ and R⁹: together form the radical =N-CH=CH-;
- alkyl: is a straight or branched saturated hydrocarbon radical having from 1 to 6 carbon atoms ; or is a cyclic saturated hydrocarbon radical having from 3 to 6 carbon atoms ; or is a a cyclic saturated hydrocarbon radical having from 3 to 6 carbon atoms attached to a straight or branched saturated hydrocarbon radical having from 1 to 6 carbon atoms ; wherein each carbon atom can be optionally substituted with halo, hydroxy, alkyloxy or oxo ;
- Ar: is a homocycle selected from the group of phenyl, naphthyl, acenaphthyl, tetrahydronaphthyl, each optionally substituted with 1, 2 or 3 substituents, each substituent independently selected from the group of hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl and mono- or dialkylaminocarbonyl ;
- Het: is a monocyclic heterocycle selected from the group of *N*-phenoxypiperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl and pyridazinyl; or a bicyclic heterocycle selected from the group of quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl or benzo[1,3]dioxolyl ; each monocyclic and bicyclic heterocycle may optionally be substituted on a carbon atom with 1, 2 or 3 substituents selected from the group of halo, hydroxy, alkyl or alkyloxy ;
- halo: is a substituent selected from the group of fluoro, chloro, bromo and iodo and
- haloalkyl: is a straight or branched saturated hydrocarbon radical having from 1 to 6 carbon atoms or a cyclic saturated hydrocarbon radical having from 3 to 6 carbon atoms, wherein one or more carbonatoms are substituted with one or more halo-atoms.

The invention also relates to the use as defined hereinabove of compounds of Formula (Ia) or (Ib) wherein
- R¹: is hydrogen, halo, haloalkyl, cyano, hydroxy, Ar, Het, alkyl, alkyloxy, alkylthio, alkyloxyalkyl, alkylthioalkyl, Ar-alkyl or di(Ar)alkyl ;
- p: is an integer equal to 1, 2, 3 or 4 ;
- R²: is hydrogen, hydroxy, mercapto, alkyloxy, alkyloxyalkyloxy, alkylthio, mono or di(alkyl)amino or a radical of formula 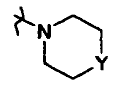 wherein Y is CH₂, O, S, NH or *N*-alkyl ;
- R³: is alkyl, Ar, Ar-alkyl, Het or Het-alkyl;
- q: is an integer equal to zero, 1, 2, 3 or 4 ;
- R⁴ and R⁵: each independently are hydrogen, alkyl or benzyl; or
- R⁴ and R⁵: together and including the N to which they are attached may form a radical selected from the group of pyrrolidinyl, 2*H*-pyrrolyl, 2-pyrrolinyl, 3-pyrrolinyl, pyrrolyl, imidazolidinyl, pyrazolidinyl, 2-imidazolinyl, 2-pyrazolinyl, imidazolyl, pyrazolyl, triazolyl, piperidinyl, pyridinyl, piperazinyl, imidazolidinyl, pyridazinyl, pyrimidinyl, pyrazinyl, triazinyl, morpholinyl and thiomorpholinyl, optionally substituted with alkyl, halo, haloalkyl, hydroxy, alkyloxy, amino, mono- or dialkylamino, alkylthio, alkyloxyalkyl, alkylthioalkyl and pyrimidinyl ;
- R⁶: is hydrogen, halo, haloalkyl, hydroxy, Ar, alkyl, alkyloxy, alkylthio, alkyloxyalkyl, alkylthioalkyl Ar-alkyl or di(Ar)alkyl ;
- r: is an integer equal to 1,2,3,4 or 5 ; and
- R⁷: is hydrogen, alkyl, Ar or Het;
- R⁸: is hydrogen or alkyl ;
- R⁹: is oxo; or
- R⁸ and R⁹: together form the radical =N-CH=CH-;
- alkyl: is a straight or branched saturated hydrocarbon radical having from 1 to 6 carbon atoms; or is a cyclic saturated hydrocarbon radical having from 3 to 6 carbon atoms; or is a a cyclic saturated hydrocarbon radical having from 3 to 6 carbon atoms attached to a straight or branched saturated hydrocarbon radical having from 1 to 6 carbon atoms ; wherein each carbon atom can be optionally substituted with halo, hydroxy, alkyloxy or oxo ;
- Ar: is a homocycle selected from the group of phenyl, naphthyl, acenaphthyl, tetrahydronaphthyl, each optionally substituted with 1, 2 or 3 substituents, each substituent independently selected from the group of hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl and mono- or dialkylaminocarbonyl ;
- Het: is a monocyclic heterocycle selected from the group of *N*-phenoxypiperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl and pyridazinyl; or a bicyclic heterocycle selected from the group of quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl or benzo[1,3]dioxolyl ; each monocyclic and bicyclic heterocycle may optionally be substituted on a carbon atom with 1, 2 or 3 substituents selected from the group of halo, hydroxy, alkyl or alkyloxy ;
- halo: is a substituent selected from the group of fluoro, chloro, bromo and iodo and
- haloalkyl: is a straight or branched saturated hydrocarbon radical having from 1 to 6 carbon atoms or a cyclic saturated hydrocarbon radical having from 3 to 6 carbon atoms, wherein one or more carbon atoms are substituted with one or more halo-atoms.

The invention also relates to the use as defined hereinabove of compounds of Formula (Ia) or (Ib) wherein :
- R¹: is hydrogen, halo, cyano, Ar, Het, alkyl, and alkyloxy ;
- p: is an integer equal to zero, 1, 2, 3 or 4 ;
- R²: is hydrogen, hydroxy, alkyloxy, alkyloxyalkyloxy, alkylthio or a radical of formula 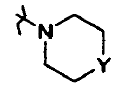 wherein Y is O ;
- R³: is alkyl, Ar, Ar-alkyl or Het ;
- q: is an integer equal to zero, 1, 2, or 3 ;
- R⁴ and R⁵: each independently are hydrogen, alkyl or benzyl; or
- R⁴ and R⁵: together and including the N to which they are attached may form a radical selected from the group of pyrrolidinyl, imidazolyl, triazolyl, piperidinyl, piperazinyl, pyrazinyl,morpholinyl and thiomorpholinyl, optionally substituted with alkyl and pyrimidinyl;
- R⁶: is hydrogen, halo or alkyl ; or
- two vicinal R⁶: radicals may be taken together to form a bivalent radical of formula -CH=CH-CH=CH- ;
- r: is an integer equal to 1 ; and
- R⁷: is hydrogen ;
- R⁸: is hydrogen or alkyl ;
- R⁹: is oxo ; or
- R⁸ and R⁹: together form the radical =N-CH=CH-.
- alkyl: is a straight or branched saturated hydrocarbon radical having from 1 to 6 carbon atoms ; or is a cyclic saturated hydrocarbon radical having from 3 to 6 carbon atoms ; or is a a cyclic saturated hydrocarbon radical having from 3 to 6 carbon atoms attached to a straight or branched saturated hydrocarbon radical having from 1 to 6 carbon atoms ; wherein each carbon atom can be optionally substituted with halo or hydroxy ;
- Ar: is a homocycle selected from the group of phenyl, naphthyl, acenaphthyl, tetrahydronaphthyl, each optionally substituted with 1, 2 or 3 substituents, each substituent independently selected from the group of halo, haloalkyl, cyano, alkyloxy and morpholinyl ;
- Het: is a monocyclic heterocycle selected from the group of *N*-phenoxypiperidinyl, furanyl, thienyl, pyridinyl, pyrimidinyl ; or a bicyclic heterocycle selected from the group of benzothienyl, 2,3-dihydrobenzo[1,4]dioxinyl or benzo[1,3]-dioxolyl; each monocyclic and bicyclic heterocycle may optionally be substituted on a carbon atom with 1, 2 or 3 alkyl substituents ; and
- halo: is a substituent selected from the group of fluoro, chloro and bromo.

For compounds according to either Formula (Ia) and (Ib), preferably, R¹ is hydrogen, halo, Ar, alkyl or alkyloxy. More preferably, R¹ is halo. Most preferably, R¹ is bromo.

Preferably, p is equal to 1.

Preferably, R² is hydrogen, alkyloxy or alkylthio. More preferably, R² is alkyloxy, in particular C₁₋₄alkyloxy. Most preferably, R² is methyloxy.

C₁₋₄alkyl is a straight or branched saturated hydrocarbon radical having from 1 to 4 carbon atoms such as for example methyl, ethyl, propyl, 2-methyl-ethyl and the like.

Preferably, R³ is naphthyl, phenyl or thienyl, each optionally substituted with 1 or 2 substituents, that substituent preferably being a halo or haloalkyl, most preferably being a halo. More preferably, R³ is naphthyl or phenyl, each optionally substituted with halo, preferably 3-fluoro. Even more preferably, R³ is naphthyl or phenyl. Most preferably, R³ is naphthyl.

Preferably, q is equal to zero, 1 or 2. More preferably, q is equal to 1.

Preferably, R⁴ and R⁵ each independently are hydrogen or alkyl, in particular hydrogen or C₁₋₄alkyl, more in particular C₁₋₄alkyl, more preferably hydrogen, methyl or ethyl, most preferably methyl.
C₁₋₄alkyl is a straight or branched saturated hydrocarbon radical having from 1 to 4 carbon atoms such as for example methyl, ethyl, propyl, 2-methyl-ethyl and the like.

Preferably R⁴ and R⁵ together and including the N to which they are attached form a radical selected from the group of imidazolyl, triazolyl, piperidinyl, piperazinyl and thiomorpholinyl, optionally substituted with alkyl, halo, haloalkyl, hydroxy, alkyloxy, alkylthio, alkyloxyalkyl or alkylthioalkyl, preferably substituted with alkyl, most preferably substituted with methyl or ethyl.

Preferably, R⁶ is hydrogen, alkyl or halo. Most preferably, R⁶ is hydrogen. Preferably r is 0, 1 or 2.

Preferably, R⁷ is hydrogen or methyl, more preferably hydrogen.

For compounds according to Formula (Ib) only, preferably, R⁸ is alkyl, preferably methyl and R⁹ is oxygen.

An interesting group of compounds are the compounds according to formula (Ia), the pharmaceutically acceptable acid or base addition salts thereof, the stereochemically isomeric forms thereof, the tautomeric forms thereof or the *N*-oxide forms thereof.

An interesting group of compounds are the compounds according to Formula (Ia), the pharmaceutically acceptable acid or base addition salts thereof, the stereochemically isomeric forms thereof, the tautomeric forms thereof or the *N*-oxide forms thereof, in which R¹ is hydrogen, halo, Ar, alkyl or alkyloxy, p = 1, R² is hydrogen, alkyloxy or alkylthio, R³ is naphthyl, phenyl or thienyl, each optionally substituted with 1 or 2 substituents selected from the group of halo and haloalkyl, q = 0, 1, 2 or 3, R⁴ and R⁵ each independently are hydrogen or alkyl or R⁴ and R⁵ together and including the N to which they are attached form a radical selected from the group of imidazolyl, triazolyl, piperidinyl, piperazinyl and thiomorpholinyl, R⁶ is hydrogen, alkyl or halo, r is equal to 0 or 1 and R⁷ is hydrogen.

Preferable, the compound is :
o 1-(6-bromo-2-methoxy-quinolin-3-yl)-2-(3,5-difluoro-phenyl)-4-dimethylamino-1-phenyl-butan-2-ol;
o 1-(6-bromo-2-methoxy-quinolin-3-yl)-4-dimethylamino-2-naphthalen-1-yl-1-phenyl-butan-2-ol corresponding to 6-bromo-α-[2-(dimethylamino)ethyl]-2-methoxy-α-1-naphthalenyl-β-phenyl-3-quinolineethanol;
o 1-(6-bromo-2-methoxy-quinolin-3-yl)-2-(2,5-difluoro-phenyl)-4-dimethylamino-1-phenyl-butan-2-ol ;
o 1-(6-bromo-2-methoxy-quinolin-3-yl)-2-(2,3-difluoro-phenyl)-4-dimethylamino-1-phenyl-butan-2-ol ;
o 1-(6-bromo-2-methoxy-quinolin-3-yl)-4-dimethylamino-2-(2-fluoro-phenyl)-1-phenyl-butan-2-ol;
o 1-(6-bromo-2-methoxy-quinolin-3-yl)-4-dimethylamino-2-naphthalen-1-yl-1-p-tolyl-butan-2-ol ;
o 1-(6-bromo-2-methoxy-quinolin-3-yl)-4-methylarnino-2-naphthalen-l-yl-1-phenyl-butan-2-ol;
o 1-(6-bromo-2-methoxy-qumolin-3-yl)-4-dimethylamino-2-(3-fluoro-phenyl)-1-phenyl-butan-2-ol; and
o 1-(6-bromo-2-methoxy-quinolin-3-yl)-4-dimethylamino-2-phenyl-1-phenyl-butan-2-ol,
a pharmaceutically acceptable acid or base addition salt thereof, a stereochemically isomeric form thereof, a tautomeric form thereof or a *N*-oxide form thereof.

Even more preferably, the compound is
o 1-(6-bromo-2-methoxy-quinolin-3-yl)-4-dimethylamino-2-(3-fluoro-phenyl)-1-phenyl-butan-2-ol;
o 1-(6-bromo-2-methoxy-quinolin-3-yl)-4-dimethylamino-2-phenyl-1-phenyl-butan-2-ol;
o 1-(6-bromo-2-methoxy-quinolin-3-yl)-4-dimethylamino-2-naphthalen-1-yl-1-phenyl-butan-2-ol corresponding to 6-bromo-α-[2-(dimethylamino)ethyl]-2-methoxy-α-1-naphthalenyl-β-phenyl-3-quinolineethanol;
a pharmaceutically acceptable acid or base addition salt thereof, a stereochemically isomeric form thereof, a tautomeric form thereof or a *N*-oxide form thereof.

An alternative chemical name for 1-(6-bromo-2-methoxy-quinolin-3-yl)-4-dimethylamino-2-naphthalen-1-yl-1-phenyl-butan-2-ol is 6-bromo-α-[2-(dimethylamino)ethyl]-2-methoxy-α-1-naphthalenyl-β-phenyl-3-quinolineethanol. Said compound can also be represented as follows:

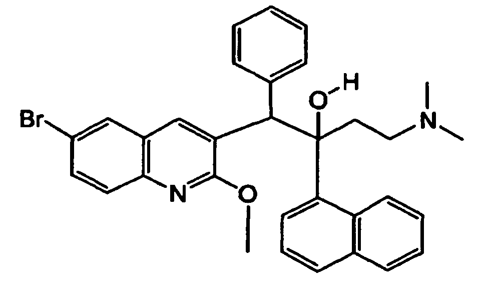

Most preferably, the compound is one of the following:
6-bromo-α-[2-(dimethylamino)ethyl]-2-methoxy-α-1-naphthalenyl-β-phenyl-3-quinolineethanol, a pharmaceutically acceptable acid or base addition salt thereof, a stereochemically isomeric forms thereof, a tautomeric form thereof or a *N*-oxide form thereof; or
6-bromo-α-[2-(dimethylamino)ethyl]-2-methoxy-α-1-naphthalenyl-β-phenyl-3-quinolineethanol, or a pharmaceutically acceptable acid addition salt thereof or 6-bromo-α-[2-(dimethylamino)ethyl]-2-methoxy-α-1-naphthalenyl-β-phenyl-3-quinolineethanol, or a stereochemically isomeric form thereof; or
6-bromo-α-[2-(dimethylamino)ethyl]-2-methoxy-α-1-naphthalenyl-β-phenyl-3-quinolineethanol, or a *N*-oxide form thereof; or
(αS, βR)-6-bromo-α-[2-(dimethylamino)ethyl]-2-methoxy-α-1-naphthalenyl-β-phenyl-3-quinolineethanol, i.e. compound 12, or a pharmaceutically acceptable acid addition salt thereof; or
(αS, βR)-6-bromo-α-[2-(dimethylamino)ethyl]-2-methoxy-α-1-naphthalenyl-β-phenyl-3-quinolineethanol, i.e. compound 12.

Thus, most preferably, the compound is (αS, βR)-6-bromo-α-[2-(dimethylamino)ethyl]-2-methoxy-α-1-naphthalenyl-β-phenyl-3-quinolineethanol which corresponds to (1R,2S)-1-(6-bromo-2-methoxy-quinolin-3-yl)-4-dimethylamino-2-naphthalen-1-yl-1-phenyl-butan-2-ol. Said compound can also be represented as follows:

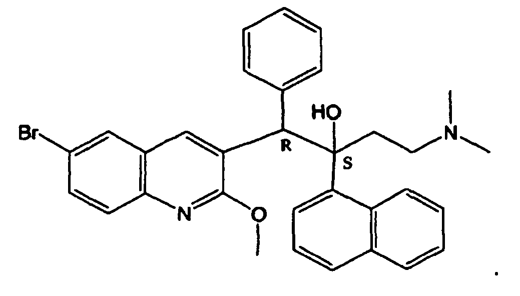

The pharmaceutically acceptable acid addition salts are defined to comprise the therapeutically active non-toxic acid addition salt forms which the compounds according to either Formula (Ia) and (Ib) are able to form. Said acid addition salts can be obtained by treating the base form of the compounds according to either Formula (Ia) and (Ib) with appropriate acids, for example inorganic acids, for example hydrohalic acid, in particular hydrochloric acid, hydrobromic acid, sulfuric acid, nitric acid and phosphoric acid ; organic acids, for example acetic acid, hydroxyacetic acid, propanoic acid, lactic acid, pyruvic acid, oxalic acid, malonic acid, succinic acid, maleic acid, furnaric acid, malic acid, tartaric acid, citric acid, methanesulfonic acid, ethanesulfonic acid, benzenesulfonic acid, p-toluenesulfonic acid, cyclamic acid, salicyclic acid, p-aminosalicylic acid and pamoic acid.

The compounds according to either Formula (Ia) and (Ib) containing acidic protons may also be converted into their therapeutically active non-toxic base addition salt forms by treatment with appropriate organic and inorganic bases. Appropriate base salts forms comprise, for example, the ammonium salts, the alkaline and earth alkaline metal salts, in particular lithium, sodium, potassium, magnesium and calcium salts, salts with organic bases, e.g. the benzathine, *N*-methyl-D-glucamine, hybramine salts, and salts with amino acids, for example arginine and lysine.

Conversely, said acid or base addition salt forms can be converted into the free forms by treatment with an appropriate base or acid.

The term addition salt as used in the framework of this application also comprises the solvates which the compounds according to either Formula (Ia) and (Ib) as well as the salts thereof, are able to form. Such solvates are, for example, hydrates and alcoholates.

The term "stereochemically isomeric forms" as used herein defines all possible isomeric forms which the compounds of either Formula (Ia) and (Ib) may possess. Unless otherwise mentioned or indicated, the chemical designation of compounds denotes the mixture of all possible stereochemically isomeric forms, said mixtures containing all diastereomers and enantiomers of the basic molecular structure. More in particular, stereogenic centers may have the R- or S-configuration; substituents on bivalent cyclic (partially) saturated radicals may have either the cis- or trans-configuration. Stereochemically isomeric forms of the compounds of either Formula (Ia) and (Ib) are obviously intended to be embraced.

Following CAS-nomenclature conventions, when two stereogenic centers of known absolute configuration are present in a molecule, an R or S descriptor is assigned (based on Cahn-Ingold-Prelog sequence rule) to the lowest-numbered chiral center, the reference center. The configuration of the second stereogenic center is indicated using relative descriptors [*R**,*R**] or [*R**,*S**], where *R** is always specified as the reference center and [*R**,*R**] indicates centers with the same chirality and [*R**,*S**] indicates centers of unlike chirality. For example, if the lowest-numbered chiral center in the molecule has an S configuration and the second center is *R*, the stereo descriptor would be specified as *S*-[*R**,*S**]. If "α" and "β" are used : the position of the highest priority substituent on the asymmetric carbon atom in the ring system having the lowest ring number, is arbitrarily always in the "α" position of the mean plane determined by the ring system. The position of the highest priority substituent on the other asymmetric carbon atom in the ring system relative to the position of the highest priority substituent on the reference atom is denominated "α", if it is on the same side of the mean plane determined by the ring system, or "β", if it is on the other side of the mean plane determined by the ring system.

Compounds of either Formula (Ia) and (Ib) and some of the intermediate compounds invariably have at least two stereogenic centers in their structure which may lead to at least 4 stereochemically different structures.

The tautomeric forms of the compounds of either Formula (Ia) and (Ib) are meant to comprise those compounds of either Formula (Ia) and (Ib) wherein e.g. an enol group is converted into a keto group (keto-enol tautomerism).

The *N*-oxide forms of the compounds according to either Formula (Ia) and (Ib) are meant to comprise those compounds of either Formula (Ia) and (Ib) wherein one or several tertiary nitrogen atoms are oxidized to the so-called *N*-oxide.

The compounds of either Formula (Ia) and (Ib) as prepared in the processes described below may be synthesized in the form of racemic mixtures of enantiomers which can be separated from one another following art-known resolution procedures. The racemic compounds of either Formula (Ia) and (Ib) may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of either Formula (Ia) and (Ib) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials.

The invention also comprises derivative compounds (usually called "pro-drugs") of the pharmacologically-active compounds according to the invention, which are degraded *in vivo* to yield the compounds according to the invention. Pro-drugs are usually (but not always) of lower potency at the target receptor than the compounds to which they are degraded. Pro-drugs are particularly useful when the desired compound has chemical or physical properties that make its administration difficult or inefficient. For example, the desired compound may be only poorly soluble, it may be poorly transported across the mucosal epithelium, or it may have an undesirably short plasma half-life. Further discussion on pro-drugs may be found in Stella, V. J. et al., "Prodrugs", Drag Delivery Systems, 1985, pp. 112-176, and Drugs, 1985, 29, pp. 455-473.

Pro-drugs forms of the pharmacologically-active compounds according to the invention will generally be compounds according to either Formula (Ia) and (Ib), the pharmaceutically acceptable acid or base addition salts thereof, the stereochemically isomeric forms thereof, the tautomeric forms thereof and the *N*-oxide forms thereof, having an acid group which is esterified or amidated. Included in such esterified acid groups are groups of the formula -COOR^{x}, where R^{x} is a C₁₋₆alkyl, phenyl, benzyl or one of the following groups :

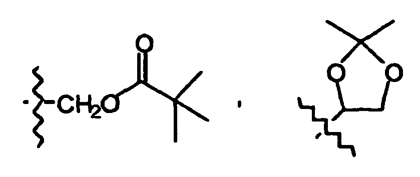

Amidated groups include groups of the formula - CONR^{y}R^{z}, wherein R^{y} is H, C₁₋₆alkyl, phenyl or benzyl and R^{z} is -OH, H, C₁₋₆alkyl, phenyl or benzyl.

Compounds according to the invention having an amino group may be derivatised with a ketone or an aldehyde such as formaldehyde to form a Mannich base. This base will hydrolyze with first order kinetics in aqueous solution.

An interesting embodiment of the present invention is the use of a substituted quinoline derivative according to Formula (Ia) or Formula (Ib), in particular (αS, βR)-6-bromo-α-[2-(dimethylamino)ethyl]-2-methoxy-α-1-naphthalenyl-β-phenyl-3-quinolineethanol, for the preparation of a medicament for the treatment of an infection with a drug resistant Mycobacterium strain as defined hereinabove wherein the drug resistant Mycobacterium strain is a drug resistant *M. tuberculosis* strain.

A further interesting embodiment of the present invention is the use of a substituted quinoline derivative according to Formula (Ia) or Formula (Ib), in particular (αS, βR)-6-bromo-α-[2-(dimethylamino)ethyl]-2-methoxy-α-1-naphthalenyl-β-phenyl-3-quinolineethanol, for the preparation of a medicament for the treatment of a human infected with a drug resistant Mycobacterium strain, in particular a drug resistant *M. tuberculosis* strain.

Still a further interesting embodiment of the present invention is the use of a substituted quinoline derivative according to Formula (Ia) or Formula (Ib), in particular (αS, βR)-6-bromo-α-[2-(dimethylamino)ethyl]-2-methoxy-α-1-naphthalenyl-β-phenyl-3-quinolineethanol, for the preparation of a medicament for the treatment of an infection with a multi drug resistant Mycobacterium strain, in particular a multi drug resistant *M. tuberculosis* strain, in particular for the preparation of a medicament for the treatment of a mammal, including a human, infected with a multi drug resistant Mycobacterium strain, in particular a multi drug resistant *M. tuberculosis* strain.

As already stated above, the compounds of formula (Ia) and (Ib) can be used to treat drug resistant including multi drug resistant Mycobacterial diseases. The exact dosage and frequency of administration depends on the particular compound of formula (Ia) or (Ib) used, the particular condition being treated, the severity of the condition being treated, the age, weight and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention.

Given the fact that the compounds of formula (Ia) and (Ib) are active against drug resistant including multi drug resistant Mycobacterial strains, the present compounds may be combined with other antimycobacterial agents in order to effectively combat Mycobacterial diseases.

Therefore, the present invention also relates to a combination of (a) a compound of formula (Ia) or (Ib), in particular (αS, βR)-6-bromo-α-[2-(dimethylamino)ethyl]-2-methoxy-α-1-naphthalenyl-β-phenyl-3-quinolineethanol or a pharmaceutically acceptable acid addition salt thereof, and (b) one or more other antimycobacterial agents.

The present invention also relates to a combination of (a) a compound of formula (Ia) or (Ib), in particular (αS, βR)-6-bromo-α-[2-(dimethylamino)ethyl]-2-methoxy-α-1-naphthalenyl-β-phenyl-3-quinolineethanol or a pharmaceutically acceptable acid addition salt thereof, and (b) one or more other antimycobacterial agents for use as a medicine.

A pharmaceutical composition comprising a pharmaceutically acceptable carrier and, as active ingredient, a therapeutically effective amount of (a) a compound of formula (Ia) or (Ib), in particular (αS, βR)-6-bromo-α-[2-(dimethylamino)ethyl]-2-methoxy-α-1-naphthalenyl-β-phenyl-3-quinolineethanol or a pharmaceutically acceptable acid addition salt thereof, and (b) one or more other antimycobacterial agents, is also comprised by the present invention.

The present invention also relates to the use of a combination or pharmaceutical composition as defined above for the treatment of an infection with a drug resistant Mycobacterium strain, in particular a drug resistant *M tuberculosis* strain.
The above defined combination or pharmaceutical composition may also be used to treat an infection with a susceptible Mycobacterial strain, in particular a susceptible *M. tuberculosis* strain.

In the above defined combination or pharmaceutical composition, the compound of formula (Ia) or (Ib) is preferably a compound of formula (Ia).

The other Mycobacterial agents which may be combined with the compounds of formula (Ia) or (Ib) are for example rifampicin (=rifampin); isoniazid; pyrazinamide; amikacin; ethionamide; moxifloxacin; ethambutol; streptomycin; para-aminosalicylic acid; cycloserine; capreomycin; kanamycin; thioacetazone; PA-824; quinolones/fluoroquinolones such as for example ofloxacin, ciprofloxacin, sparfloxacin; macrolides such as for example clarithromycin, clofazimine, amoxycillin with clavulanic acid; rifamycins; rifabutin; rifapentine.

Preferably, the present compounds of formula (Ia) or (Ib), in particular (αS, βR)-6-bromo-α-[2-(dimetbylamino)ethyl]-2-methoxy-α-1-naphthalenyl-β-phenyl-3-quinolineethanol, are combined with rifapentin and moxifloxacin.

Another interesting combination according to the present invention is a combination of (a) a compound of formula (Ia) or (Ib), in particular (αS, βR)-6-bromo-α-[2-(dimethylamino)ethyl]-2-methoxy-α-1-naphthalenyl-β-phenyl-3-quinolineethanol or a pharmaceutically acceptable acid addition salt thereof, and (b) one or more other antimycobacterial agents wherein said one or more other antimycobacterial agents comprise pyrazinamide. Thus, the present invention also relates to a combination of a compound of formula (Ia) or (Ib), in particular (αS, βR)-6-bromo-α-[2-(dimethylamino)ethyl]-2-methoxy-α-1-naphthalenyl-β-phenyl-3-quinolineethanol or a pharmaceutically acceptable acid addition salt thereof, and pyrazinamide and optionally one or more other antimycobacterial agents. Examples of such combinations are the combination of (αS, βR)-6-bromo-α-[2-(dimethylamino)ethyl]-2-methoxy-α-1-naphthalenyl-β-phenyl-3-quinolineethanol or a pharmaceutically acceptable acid addition salt thereof, and pyrazinamide; the combination of (αS, βR)-6-bromo-α-[2-(dimethylamino)ethyl]-2-methoxy-α-1-naphthalenyl-β-phenyl-3-quinolineethanol or a pharmaceutically acceptable acid addition salt thereof, pyrazinamide and rifapentin; the combination of (αS, βR)-6-bromo-α-[2-(dimethylamino)ethyl]-2-methoxy-α-1-naphthalenyl-β-phenyl-3-quinolineethanol or a pharmaceutically acceptable acid addition salt thereof, pyrazinamide and isoniazid; the combination of (αS, βR)-6-bromo-α-[2-(dimethylamino)ethyl]-2-methoxy-α-1-naphthalenyl-β-phenyl-3-quinolineethanol or a pharmaceutically acceptable acid addition salt thereof, pyrazinamide and moxifloxacin; the combination of (αS, βR)-6-bromo-α-[2-(dimethylamino)ethyl]-2-metboxy-α-1-naphthalenyl-β-phenyl-3-quinolineethanol or a pharmaceutically acceptable acid addition salt thereof, pyrazinamide and rifampin. It has been found that a compound of formula (Ia) or (Ib), in particular (αS, βR)-6-bromo-α-[2-(dimethylamino)ethyl]-2-methoxy-α-1-naphthalenyl-β-phenyl-3-quinolineethanol or a pharmaceutically acceptable acid addition salt thereof, and pyrazinamide act synergistically.

Also interesting combinations are those combinations comprising a compound of formula (Ia) or (Ib), as described in Tables 11 and 12.

A pharmaceutical composition comprising a pharmaceutically acceptable carrier and, as active ingredient, a therapeutically effective amount of the active ingredients listed in the above combinations, is also comprised by the present invention.

The present pharmaceutical composition may have various pharmaceutical forms for administration purposes. As appropriate compositions there may be cited all compositions usually employed for systemically administering drugs. To prepare the pharmaceutical compositions of this invention, an effective amount of the particular compounds, optionally in addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, in particular, for administration orally or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, diluents, lubricants, binders, disintegrating agents and the like in the case of powders, pills, capsules and tablets. Because of their ease in administration, tablets and capsules represent the most advantageous oral unit dosage forms in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations which are intended to be converted, shortly before use, to liquid form preparations.

Depending on the mode of administration, the pharmaceutical composition will preferably comprise from 0.05 to 99 % by weight, more preferably from 0.1 to 70 % by weight of the active ingredients, and, from 1 to 99.95 % by weight, more preferably from 30 to 99.9 weight % of a pharmaceutically acceptable carrier, all percentages being based on the total composition.

The weight to weight ratio's of the compound of formula (Ia) or (Ib) and (b) the other antimycobacterial agent(s) when given as a combination may be determined by the person skilled in the art. Said ratio and the exact dosage and frequency of administration depends on the particular compound of formula (Ia) or (Ib) and the other antimycobacterial agent(s) used, the particular condition being treated, the severity of the condition being treated, the age, weight and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention.

The compounds of formula (Ia) or (Ib) and the one or more other antimycobacterial agents may be combined in a single preparation or they may be formulated in separate preparations so that they can be administered simultaneously, separately or sequentially. Thus, the present invention also relates to a product containing (a) a compound of formula (Ia) or (Ib), and (b) one or more other antimycobacterial agents, as a combined preparation for simultaneous, separate or sequential use in the treatment of mycobacterial diseases.

The pharmaceutical composition may additionally contain various other ingredients known in the art, for example, a lubricant, stabilising agent, buffering agent, emulsifying agent, viscosity-regulating agent, surfactant, preservative, flavouring or colorant.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and the like, and segregated multiples thereof.
The daily dosage of the compound according to the invention will, of course, vary with the compound employed, the mode of administration, the treatment desired and the mycobacterial disease indicated. However, in general, satisfactory results will be obtained when the compound according to the invention is administered at a daily dosage not exceeding 1 gram, e.g. in the range from 10 to 50 mg/kg body weight.

The compounds of formula (Ia) and (Ib) and their preparation is described in WO 2004/011436.

Of some compounds the absolute stereochemical configuration of the stereogenic carbon atom(s) therein was not experimentally determined. In those cases the stereochemically isomeric form which was first isolated is designated as "A" and the second as "B", without further reference to the actual stereochemical configuration. However, said "A" and "B" isomeric forms can be unambiguously characterized by a person skilled in the art, using art-known methods such as, for example, X-ray diffraction.
In case "A" and "B" are stereoisomeric mixtures, they can be further separated whereby the respective first fractions isolated are designated "A1" and "B1" and the second as "A2" and "B2", without further reference to the actual stereochemical configuration.

The following Tables list compounds of formula (Ia) and (Ib), which can all be prepared according to the methods described in WO 2004/011436.

**Table 1 :**

| | | | | | | |
|---|---|---|---|---|---|---|
| 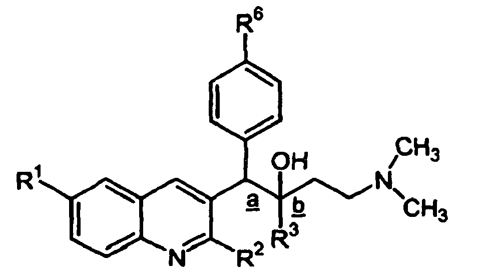 | | | | | | |

| **Comp. nr.** | **Ex. nr.** | **R¹** | **R²** | **R³** | **R⁶** | **Stereochemistry and melting points** |
|---|---|---|---|---|---|---|
| 1 | B1 | Br | OCH₃ | phenyl | H | (A1); 194°C |
| 2 | B1 | Br | OCH₃ | phenyl | H | (A2); 191°C |
| 3 | B1 | Br | OCH₃ | phenyl | H | (A);200°C |
| 4 | B1 | Br | OCH₃ | phenyl | H | (B); 190°C |
| 16 | B1 | Br | OCH₃ | 4-chlorophenyl | H | (A); 200°C |
| 17 | B1 | Br | OCH₃ | 4-chlorophenyl | H | (B); 190°C |
| 20 | B1 | Br | OCH₃ | 2-thienyl | H | (A); 96°C |
| 21 | B1 | Br | OCH₃ | 2-thienyl | H | (B); 176°C |
| 22 | B1 | CH₃ | OCH₃ | phenyl | H | (A); 148°C |
| 23 | B1 | CH₃ | OCH₃ | phenyl | H | (B); 165°C |
| 24 | B1 | Br | OCH₃ | 3-thienyl | H | (A); 162°C |
| 25 | B1 | Br | OCH₃ | 3-thienyl | H | (B); 160°C |
| 26 | B1 | phenyl | OCH₃ | phenyl | H | (A); 174°C |
| 27 | B1 | phenyl | OCH₃ | phenyl | H | (B); 192°C |
| 28 | B1 | F | OCH₃ | phenyl | H | (A); 190°C |
| 29 | B1 | F | OCH₃ | phenyl | H | (B); 166°C |
| 30 | B1 | Cl | OCH₃ | phenyl | H | (A); 170°C |
| 31 | B1 | Cl | OCH₃ | phenyl | H | (B); 181°C |
| 32 | B1 | Br | SCH₃ | phenyl | H | (A); 208°C |
| 33 | B1 | Br | SCH₃ | phenyl | H | (B); 196°C |
| 34 | B1 | OCH₃ | OCH₃ | pheny | H | (A); 165°C |
| 35 | B1 | OCH₃ | OCH₃ | phenyl | H | (B); 165°C |
| 36 | B1 | Br | OCH₃ | phenyl | Cl | (A); 197°C |
| 37 | B1 | Br | OCH₃ | phenyl | Cl | (B); 221°C |
| 38 | B9 | Br | OCH₃ | 3-fluorophenyl | H | (A); 198°C |
| 39 | B9 | Br | OCH₃ | 3-fluomphenyl | H | (B); 207°C |
| 108 | B9 | Br | OCH₃ | 3-fluorophenyl | H | (A1); 160°C |
| 109 | B9 | Br | OCH₃ | 3-fluorophenyl | H | (A2); 156°C |
| 40 | B1 | H | OCH₃ | phenyl | H | (A); 152°C |
| 41 | B1 | H | OCH₃ | phenyl | H | (B); 160°C |
| 42 | B1 | H | OCH₃ | CH₃ | H | (A); 140°C |
| 43 | B1 | H | OCH₃ | CH₃ | H | (B); 120°C |
| 59 | B1 | Br | OH | phenyl | H | (A); >260°C |
| 60 | B1 | Br | OH | phenyl | H | (B); 215°C |
| 5 | B2 | Br | OCH₂CH₃ | phenyl | H | (A); 162°C |
| 6 | B2 | Br | OCH₂CH₃ | phenyl | H | (B); 74°C |
| 7 | B3 | Br | H | phenyl | H | (A); 98°C |
| 8 | B3 | Br | H | phenyl | H | (B); 180°C |
| 12 | B7 | Br | OCH₃ | 1-naphthyl | H | (A1); 118°C; a=R, b=S; [alpha]_{D}²⁰ = -166.98 (c=0.505g/100ml in DMF) |
| 13 | 87 | Br | OCH₃ | 1-naphthyl | H | (A2); 120°C; a=S; b=R; [alpha]_{D}²⁰ = +167.60 (c=0.472g/100ml in DMF) |
| 14 | B7 | Br | OCH₃ | 1-naphthyl | H | (A); 210°C |
| 15 | B7 | Br | OCH₃ | 1-naphthyl | H | (B); 244°C |
| 45 | B7 | Br | OCH₃ | 2-naphthyl | H | (A); 262°C |
| 46 | B7 | Br | OCH₃ | 2-naphthyl | H | (B); 162°C |
| 67 | B8 | Br | OCH₃ | 2,5-difluorophenyl | H | (A); 60°C |
| 68 | B8 | Br | OCH₃ | 2,5-difluorophenyl | H | (B); 208°C |
| 110 | B8 | Br | OCH₃ | 2,5-difluorophenyl | H | (A1); 167°C |
| 111 | B8 | Br | OCH₃ | 2,5-difluorophenyl | H | (A2); oil |
| 69 | B1 | Br | OCH₃ | 2-fluorophenyl | H | (A); oil |
| 70 | B1 | Br | OCH₃ | 2-fluorophenyl | H | (B); oil |
| 71 | B1 | Br | OCH₃ | 1-naphthyl | CH₃ | (A); 174°C |
| 72 | B1 | Br | OCH₃ | 1-naphthyl | CH₃ | (B); 178°C |
| 73 | B1 | Br | OCH₃ | 1-naphthyl | Cl | (B); 174°C |
| 74 | B1 | Br | OCH₃ | 1-naphthyl | Cl | (A); 110°C |
| 75 | B1 | Br | OCH₃ | 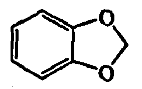 | H | (A); 196°C |
| 76 | B1 | Br | OCH₃ | 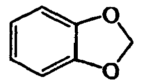 | H | (B); 130°C |
| 77 | B1 | Br | OCH₃ | 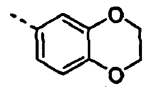 | H | (A); 202°C |
| 78 | B1 | Br | OCH₃ | 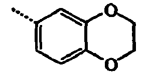 | H | (B); 202°C |
| 79 | B1 | Br | | 1-naphthyl | H | (A); >250°C |
| 80 | B1 | Br | OCH₃ | 4-cyanophenyl | H | (A); 224°C |
| 81 | B1 | Br | OCH₃ | 4-cyanophenyl | H | (B); 232°C |
| 82 | 81 | CH₃ | OCH₃ | 1-naphthyl | H | (A); 202°C |
| 83 | B1 | CH₃ | OCH₃ | 1-naphthyl | H | (B); 198°C |
| 84 | B1 | phenyl | OCH₃ | 1-naphthyl | H | (A); 248°C |
| 85 | B1 | phenyl | OCH₃ | 1-naphthyl | H | (B); 214°C |
| 86 | B1 | Br | OCH₃ | 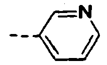 | H | (A); 184°C |
| 87 | B1 | Br | OCH₃ | 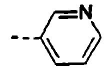 | H | (B); 186°C |
| 88 | B1 | Br | SCH₃ | 1-naphthyl | H | (A); 240°C |
| 89 | B1 | Br | OCH₃ | 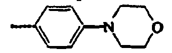 | H | (A); 236°C |
| 90 | B1 | Br | OCH₃ | 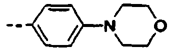 | H | (B); 206°C |
| 91 | B1 | H | OCH₃ | 1-naphthyl | H | (A); 178°C |
| 92 | B1 | H | OCH₃ | 1-naphthyl | H (B); | 160°C |
| 93 | B1 | H | OCH₃ | 3-fluoropheayl | H | (A); 178°C |
| 94 | B1 | H | OCH₃ | 3-fluorophenyl | H | (B); 182°C |
| 95 | B1 | Br | OCH₃ | 2-phenylethyl | H | (A); 178°C |
| 96 | B1 | Br | OCH₃ | 2-phenylethyl | H | (B); 146°C |
| 97 | B1 | OCH₃ | OCH₃ | 1-naphthyl | H | (A); 168°C |
| 98 | B1 | OCH₃ | OCH₃ | 1-naphthyl | H | (B); 154°C |
| 113 | B14 | Br | OCH₃ | 2,3-difluorophenyl | H | (A); 128°C |
| 114 | B14 | Br | OCH₃ | 2,3-difluorophenyl | H | (B); 213°C |
| 115 | B15 | Br | OCH₃ | 3,5-difluorophenyl | H | (A); 192°C |
| 116 | B15 | Br | OCH₃ | 3,5-difluorophenyl | H | (B); 224°C |
| 117 | B15 | Br | OCH₃ | 3,5-difluorophenyl | H | (A1); 161°C |
| 118 | B15 | Br | OCH₃ | 3,5-difluorophenyl | H | (A2); 158°C |
| 119 | B7 | Cl | OCH₃ | 1-naphthyl | H | (A); 212°C |
| 120 | B7 | Cl | OCH₃ | 1-naphthyl | H | (B); 236°C |
| 122 | B7 | Br | OCH₃ | 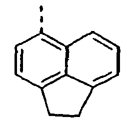 | H | (B); 227°C |
| 127 | B7 | Br | OCH₃ | 5-bromo-2-naphthyl | H | (A); 226°C |
| 130 | B7 | Br | OCH₃ | 5-bromo-2-naphthyl | H | (B); 220°C |
| 131 | B1 | Br | OCH₃ | 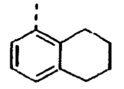 | H | (A); 206°C |
| 134 | B9 | OCH₃ | OCH₃ | 3-fluorophenyl | H | (A); 172°C |
| 135 | B9 | OCH₃ | OCH₃ | 3-fluorophenyl | H | (B); 182°C |
| 143 | B7 | Br | OCH₃ | 3-bromo-1-naphthyl | H | (A); 234°C |
| 150 | B7 | Br | OCH₃ | 3-bromo-1-naphthyl | H | (B); 212°C |
| 159 | B8 | Br | OCH₃ | 2,5-difluorophenyl | H | (A1); 208°C |
| 160 | B8 | Br | OCH₃ | 2,5-difluornphenyl | H | (A2); 167°C |
| 162 | B7 | Br | OCH₃ | 6-methoxy-2-naphthyl | H | (A); 206°C |
| 163 | B7 | Br | OCH₃ | 6-methoxy-2-naphthyl | H | (B); 206°C |
| 164 | B9 | Br | 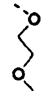 | 3-fluorophenyl | H | (A); 118°C |
| 165 | B9 | Br | 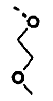 | 3-fluorophenyl | H | (B); oil |
| 167 | B8 | Br | OCH₃ | 2,6-difluorophenyl | H | (B); 180°C |
| 174 | B9 | 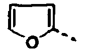 | OCH₃ | 3-fluorophenyl | H | (A); 159°C |
| 175 | B9 | 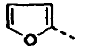 | OCH₃ | 3-fluorophcnyl | H | (B); 196°C |
| 176 | B7 | Br | 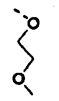 | 1-naphthyl | H | (A); oil |
| 179 | B9 | CN | OCH₃ | 3-fluorophenyl | H | (A); 213°C |
| 180 | B9 | CN | OCH₃ | 3-fluorophenyl | H | (B); 163°C |
| 181 | B9 | Br | OCH₃ | 4-fluorophenyl | H | (A); 198°C |
| 182 | B9 | Br | OCH₃ | 4-fluorophenyl | H | (B); 238°C |
| 183 | B1 | Br | OCH₃ | 3-trifluoromethylphenyl | H | (A); 170°C |
| 188 | B1 | Br | OCH₃ | 1,4-pyrimidin-2-yl | H | (A); 110°C |
| 189 | B1 | Br | OCH₃ | 1,4-pyrimidin-2-yl | H | (B); 145°C |
| 195 | B15 | Br | OCH₃ | 3,4-difluorophenyl | H | (A); 250°C |
| 196 | B15 | Br | OCH₃ | 3,4-difluorophenyl | H | (B); 184°C |
| 201 | B1 | Br | OCH₃ | 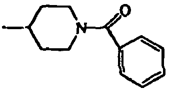 | H | (A); 214°C |
| 202 | B1 | Br | OCH₃ | 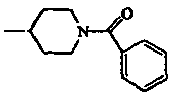 | H | (B); 246°C |
| 203 | B9 | 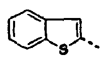 | OCH₃ | 3-fluorophenyl | H | (A); 225°C |
| 204 | B9 | 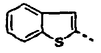 | OCH₃ | 3-fluorophenyl | H | (B); 216°C |
| 205 | B7 | Br | OCH₃ | 1-naphthyl | F | (A); 213°C |
| 206 | B7 | Br | OCH₃ | 1-naphthyl | F | (B); 213°C |
| 207 | B15 | F | OCH₃ | 3,5-difluorophenyl | H | (A); 232°C |
| 208 | B15 | F | OCH₃ | 3,5-difluorophenyl | H | (B); 188°C |
| 212 | B7 | 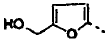 | OCH₃ | 1-naphthyl | H | (B); 220°C |

**Table 2:**

| | | | | | | | |
|---|---|---|---|---|---|---|---|
| 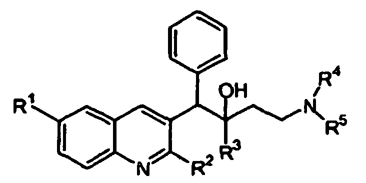 | | | | | | | |

| **Comp. nr.** | **Ex. nr.** | **R¹** | **R²** | **R³** | **R⁴** | **R⁵** | **Phys. data (salt/melting points) and stereochemistry** |
|---|---|---|---|---|---|---|---|
| 18 | B1 | Br | OCH₃ | phenyl | CH₂CH₃ | CH₂CH₃ | ethanedioate (2:3); (A); 230°C |
| 19 | B1 | Br | OCH₃ | phenyl | CH₂CH₃ | CH₂CH₃ | .ethanedioate (2:3), (B); 150°C |
| 44 | B4 | Br | OCH₃ | phenyl | H | H | (A); 190°C |
| 9 | B4 | Br | OCH₃ | phenyl | H | H | (B); 204°C |
| 141 | B7 | Br | OCH₃ | 2-naphthyl | CH₃ | CH₂CH₃ | (A); 188°C |
| 142 | B7 | Br | OCH₃ | 2-naphthyl | CH₃ | CH₂CH₃ | (B); 202°C |
| 230 | B12 | Br | OCH₃ | 1-naphthyl | CH₃ | benzyl | /oil |
| 147 | B7 | Br | OCH₃ | 1-naphthyl | CH₃ | CH₂CH₃ | (A); 168°C |
| 148 | B7 | Br | OCH₃ | 1-naphthyl | CH₃ | CH₂CH₃ | (B); 212°C |
| 56 | B13 | Br | OCH₃ | 1-naphthyl | CH₃ | H | (A); 204°C |
| 214 | B13 | Br | OCH₃ | 1-naphthyl | CH₃ | H | (B); 225°C |

**Table 3:**

| | | | | |
|---|---|---|---|---|
| 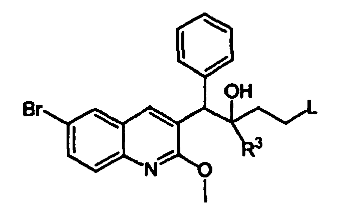 | | | | |

| **Comp. nr.** | **Ex. nr.** | **R³** | **L** | **Stereochemistry and melting points** |
|---|---|---|---|---|
| 47 | B1 | phenyl | 1-piperidinyl | (A); 190°C |
| 48 | B1 | phenyl | 1-piperidinyl | (B); 210°C |
| 128 | B1 | 2-naphthyl | 1-piperidinyl | (A); 254°C |
| 129 | B1 | 2-naphthyl | 1-piperidinyl | (B); 212°C |
| 49 | B1 | phenyl | 1-imidazolyl | (A); 216°C |
| 50 | B1 | phenyl | 1-imidazolyl | (B); 230°C |
| 51 | B1 | phenyl | 1-(4-methyl)piperazinyl | (A); 150°C |
| 52 | B1 | phenyl | 1-(4-methyl)piperazinyl | (B); 230°C |
| 53 | B1 | phenyl | 1-(1,2,4-triazolyl) | (A); 180°C |
| 54 | B1 | phenyl | 1-(1,2,4-triazolyl) | (B); 142°C |
| 55 | B1 | phenyl | thiomorpholinyl | (A); oil |
| 57 | B5 | phenyl | 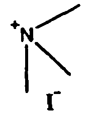 | (A); 244°C |
| 10 | B5 | phenyl | 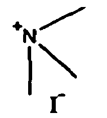 | (B); 198°C |
| 58 | B6 | phenyl | 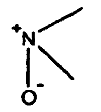 | (A); 208°C |
| 11 | B6 | phenyl | 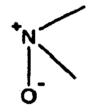 | (B); 208°C |
| 99 | B11 | 1-naphthyl | 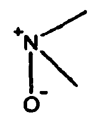 | (A1); 218°C |
| 100 | B6 | 1-naphthyl | 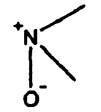 | (A2); 218°C |
| 101 | B6 | 1-naphthyl | 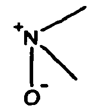 | (B); 175°C |
| 102 | B5 | 1-naphthyl | 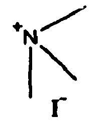 | (A2); 210°C |
| 103 | B5 | 1-naphthyl | 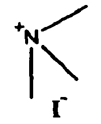 | (B); >250°C |
| 121 | B5 | 1-naphthyl | 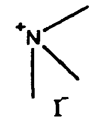 | (A1); 210°C |
| 123 | B1 | phenyl | morpholinyl | (A); 226°C |
| 124 | B1 | phenyl | morpholinyl | (B); 210°C |
| 136 | B7 | 2-naphthyl | 4-methylpyrazinyl | (A); 188°C |
| 137 | B7 | 2-naphthyl | 4-methylpyrazinyl | (B); 232°C |
| 139 | B7 | 2-naphthyl | morpholinyl | (A); 258°C |
| 140 | B7 | 2-naphthyl | morpholinyl | (B); 214°C |
| 144 | B7 | 2-naphthyl | pyrrolidinyl | (A); 238°C |
| 145 | B7 | 1-naphthyl | 1-piperidinyl | (A); 212°C |
| 146 | B7 | 1-naphthyl | 1-piperidinyt | (B); 220°C |
| 149 | B7 | 1-naphthyl | 4-methylpyrazinyl | (B); 232°C |
| 151 | B7 | 3-bromo-1-naphthyl | 4-methylpiperazinyl | (A); 178°C |
| 152 | B7 | 3-bromo-1-naphthyl | 4-methylpiperazinyl | (B); 226°C |
| 153 | B7 | 6-bromo-2-naphthyl | 4-methylpiperazinyl | (A); 208°C |
| 154 | B7 | 6-bromo-2-naphthyl | 4-methylpiperazinyl | (B); 254°C |
| 155 | B7 | 6-bromo-2-naphthyl | 1-piperidinyl | (A); 224°C |
| 156 | B7 | 1-naphthyl | 4-methylpiperazinyl | (A); 200°C |
| 157 | B7 | 6-bromo-2-naphthyl | 1-pyrrolidinyl | (B); 220°C |
| 158 | B7 | 1-naphthyl | morpholinyl | (B); 272°C |
| 166 | B7 | 6-bromo-2-naphthyl | 1-piperidinyl | (B); 218°C |
| 170 | B7 | 2-naphthyl | 1-pyrrolidinyl | (A); 238°C |
| 171 | B7 | 2-naphthyl | 1-pyrrolidinyl | (B); 218°C |
| 172 | B7 | 1-naphthyl | 1,2,4-triazol-1-yl | /142°C |
| 173 | B7 | 1-naphthyl | 1,2-imidazol-1-yl | (A); 222°C |
| 177 | B7 | 6-bromo-2-naphthyl | morpholinyl | (A); 242°C |
| 178 | B7 | 6-bromo-2-naphthyl | morpholinyl | (B); 246°C |
| 187 | B7 | 1-naphthyl | 1,2-imidazol-1-yl | (B); 236°C |
| 200 | B7 | 2-naphthyl | 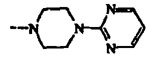 | (A); 254°C |
| 209 | B7 | 2-naphthyl | 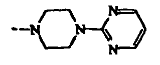 | (B); 198°C |

**Table 4:**

| | | | | | |
|---|---|---|---|---|---|
| 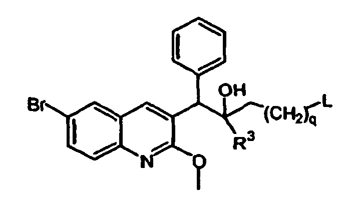 | | | | | |

| **Comp. nr.** | **Ex. nr.** | **R³** | **Q** | **L** | **Stereochemistry and melting points** |
|---|---|---|---|---|---|
| 61 | B1 | phenyl | 0 | N(CH₃)₂ | (A); 220°C |
| 62 | B1 | phenyl | 0 | N(CH₃)₂ | (B); 194°C |
| 63 | B1 | phenyl | 2 | N(CH₃)₂ | (A); 150°C |
| 64 | B1 | phenyl | 2 | N(CH₃)₂ | (B); 220°C |
| 125 | B7 | 2-naphthyl | 2 | N(CH₃)₂ | (A); 229°C |
| 126 | 87 | 2-naphthyl | 2 | N(CH₃)₂ | (B); 214°C |
| 65 | B1 | phenyl | 3 | N(₃)₂ | CH(A); 130°C |
| 66 | B1 | phenyl | 3 | N(CH₃)₂ | (B); 170°C |
| 132 | B7 | 2-naphthyl | 2 | pyrrolidinyl | (A); 227°C |
| 133 | B7 | 2-naphthyl | 2 | pyrrolidinyl | (B); 222°C |
| 161 | B7 | 2-naphthyl | 2 | morpholinyl | (B); 234°C |
| 186 | B7 | 1-naphthyl | 2 | N(CH₃)₂ | (A); 187°C |
| 190 | B7 | 2-naphthyl | 3 | N(₃)₂ | CH(A); 170°C |
| 191 | B7 | 2-naphthyl | 3 | N(CH₃)₂ | (B); 145°C |
| 192 | B7 | 2-naphthyl | 2 | N(CH₂CH₃)₂ | (A); 90°C |
| 193 | B7 | 2-naphthyl | 2 | N(CH₂CH₃)₂ | (B); 202°C |
| 194 | B7 | 1-naphthyl | 2 | pyrrolidinyl | (B); 206°C |
| 197 | B7 | 1-naphthyl | 3 | N(CH₃)₂ | (A); 160°C |
| 198 | B7 | 2-naphthyl | 2 | morpholinyl | (A); 215°C |
| 199 | B7 | 1-naphthyl | 2 | N(CH₂CH₃)₂ | (A); 185°C |
| 210 | B7 | 1-naphthyl | 2 | morpholinyl | (B); 222°C |
| 211 | B7 | 1-naphthyl | 2 | morpholinyl | (A); 184°C |

**Table 5:**

| | | | | | |
|---|---|---|---|---|---|
| 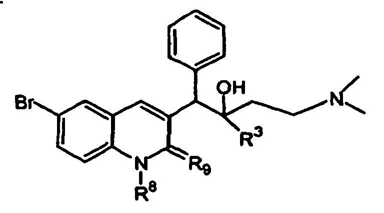 | | | | | |

| **Comp nr.** | **Ex. nr.** | **R³** | **R⁸** | **R⁹** | **Stereochemistry and melting points** |
|---|---|---|---|---|---|
| 104 | B1 | phenyl | -CH=CH-N= | | (A); 170°C |
| 105 | B1 | phenyl | -CH=CH-N= | | (B); 150°C |
| 106 | B1 | phenyl | CH₃ | =O | (A); 224°C |
| 107 | B1 | phenyl | CH₃ | =O | (B); 180°C |
| 138 | B7 | 1-naphthyl | H | =O | (A1); >260°C |

**Table 6:**

| | | | | | | | | |
|---|---|---|---|---|---|---|---|---|
| 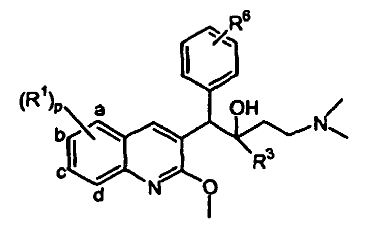 | | | | | | | | |

| **Comp. nr.** | **Ex. nr.** | **R¹** | | | | **R³** | **R⁶** | **Sterechemistry and melting points** |
|---|---|---|---|---|---|---|---|---|
| | | **a** | **b** | **c** | **d** | | | |
| 215 | B9 | H | Br | CH₃ | H | 3-fluorophenyl | H | (A); 197°C |
| 216 | B9 | H | Br | CH₃ | H | 3-fluorophenyl | H | (B); 158°C |
| 217 | B7 | H | H | Br | H | 1-naphthyl | H | (A); 212°C |
| 218 | B7 | H | H | Br | H | 1-naphthyl | H | (B); 172°C |
| 219 | B9 | H | Br | H | CH₃ | 3-fluorophenyl | H | (A); 220°C |
| 220 | B9 | H | Br | H | CH₃ | 3-fluorophenyl | H | (B); 179°C |
| 221 | B7 | Br | H | H | H | 1-naphthyl | H | (A); 110°C |
| 224 | B7 | Br | H | H | H | 1-naphthyl | H | /205°C |
| 222 | B7 | H | Br | H | H | 1-naphthyl | 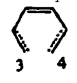 | (A); 155°C |
| 223 | B7 | H | Br | H | H | 1-naphthyl | 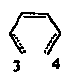 | (B); 205°C |
| 225 | B7 | H | Br | CH₃ | H | 1-naphthyl | H | (A); 238°C |
| 226 | B7 | H | Br | CH₃ | H | 1-naphthyl | H | (B); 208°C |
| 227 | B15 | H | Br | CH₃ | H | 3,5-difluorophenyl | H | (A); 195°C |
| 228 | B15 | H | Br | CH₃ | H | 3,5-difluorophenyl | H | (B); 218°C |
| 229 | B7 | H | CH₃ | CH₃ | H | 1-naphthyl | H | (A); 238°C |

### Pharmacological examples

### In-vitro method for testing compounds against resistant Mycobacteria strains.

The *in vitro* activity has been assessed by the determination of the minimal inhibitory concentration (MIC : MIC will be the lowest drug concentration inhibiting more than 99 % of the bacterial growth on control medium without antibiotic) in solid medium.

For the *in vitro* test, the following medium was used : 10 % Oleic acid Albumin Dextrose Catalase (OADC)-enriched 7H11 medium.
As inoculum was used: two appropriate dilutions of 10 % OADC-enriched 7H9 broth culture aged of 3 to 14 days depending on the mycobacterial species (final inocula = about 10² and 10⁴ cfu (colony forming units))
The incubations were done at 30°C or 37°C for 3 to 42 days depending on the mycobacterial species.

Tables 7 and 8 list the MICs (mg/L) against different clinical isolates of resistant Mycobacterium strains. Tables 9 and 10 list the MICs (mg/L) against different clinical isolates of Mycobacterium strains resistant to fluoroquinolones.
In the Tables rifampin and ofloxacin are also included as reference.

**Table 7 :**

| Strains | Rifampin | Compound 12 | Compound 109 | Compound 2 |
|---|---|---|---|---|
| *M.tuberculosis* isoniazid-resistant low level | 0.5 | 0.06 | 0.12 | 0.25 |
| *M.tuberculosis* isoniazid-resistant high level | 0.5 | ≤ 0.01 | 0.03 | ≤ 0.01 |
| *M.tuberculosis* rifampin-resistant | >256 | 0.06 | 0.12 | 0.06 |

**Table 8:**

| Strains | Rifampin | Compound 12 |
|---|---|---|
| *M.tuberculosis* isoniazid-resistant High Level | 0.25 | 0.01 |
| *M.tuberculosis* isoniazid-resistant high level | 0.5 | 0.06 |
| *M.tuberculosis* isoniazid-resistant high level | 0.12 | 0.03 |
| *M.tuberculosis* isoniazid-resistant high level | ≤ 0.06 | 0.01 |
| *M.tuberculosis isoniazid* -Resistant high level and streptomycin-resistant | 0.25 | 0.01 |
| *M. tuberculosis* rifampin-resistant | 256 | 0.03 |
| *M.tuberculosis* rifampin-resistant | 16 | 0.03 |
| *M.tuberculosis* rifampin-resistant | 256 | 0.01 |
| *M.tuberculosis* streptomycin-resistant | 0.5 | 0.01 |
| *M.tuberculosis* ethambutol-resistant | 0.25 | 0.01 |
| *M.tuberculosis* pyrazinamide-resistant | 0.5 | 0.03 |

**Table 9 :**

| Strains | Rifampin | Compound 12 | Ofloxacin |
|---|---|---|---|
| *M.tuberculosis* | 1 | 0.06 | 8 (Ala83Val Ser84Pro)* |
| *M.tuberculosis* | 2 | 0.12 | 32 (Asp87Gly)* |
| *M.avium* | 16 | 0.007 | 128 (Ala83Val)* |

| | | | |
|---|---|---|---|
| * The indications between parentheses indicate the mutations in the protein responsible for ofloxacin resistance | | | |

**Table 10 :**

| *Strains* | *Rifampin* | *Compound 12* | *Ofloxacin* |
|---|---|---|---|
| *M.smegmatis* | 64 | 0.01 | 8 (Asp87Gly)* |
| *M.smegmatis* | 64 | 0.01 | 32 (Ala 83 Val and Asp87Gly)* |
| *M.smegmatis* | 64 | 0.01 | 32 (Ala83Val and Asp87Gly)* |
| *M.smegmatis* | 128 | 0.007 | 2 (Ala83Val)* |
| *M.smegmatis* | ND | 0.003 | 32 (Asp87Gly)* |
| *M.fortuitum* | 128 | 0.01 | 1 |
| *M.fortuitum* | 128 | 0.007 | 1 (Ser84Pro)* |
| *M.fortuitum* | >64 | 0.01 | 1.5 (Asp87Gly)* |

| | | | |
|---|---|---|---|
| * The indications between parentheses indicate the mutations in the protein responsible for ofloxacin resistance. | | | |

From these results it can be concluded that the present compounds are highly active against drug resistant Mycobacterium strains. There is no evidence of cross-resistance with antituberculosis drugs : isoniazid, rifampin, streptomycin, ethambutol and pyrazinamide.
In the same manner, there is no evidence of cross-resistance with fluoroquinolones.

Compound 12 was also tested against 2 multi-drug resistant *M. tuberculosis* strains, i.e. a strain resistant to isoniazid 10 mg/L and rifampin and a strain resistant to isoniazid 0.2 mg/L and rifampin. The MIC obtained for compound 12 for both strains is 0.03mg/L.

### In vivo method for testing combinations against M. tuberculosis infected mice.

Four weeks old Swiss female mice were infected intravenously with 5x10⁶ CFU of *M.tuberculosis* H37Rv strain. On D1 and D14 following the infection, ten mice were sacrificed to determine the baseline values of spleen weight and CFU counts in the spleens and the lungs after inoculation and at the beginning of treatment. The remaining mice were allocated to the following treatment groups : an untreated control group for survival monitoring, two positive control groups, one with a regimen for susceptible tuberculosis treated with 2 months of isoniazid 25 mg/kg, rifampin 10 mg/kg, pyrazinamide 150 mg/kg daily, and the other with a regimen for multi drug resistant tuberculosis treated with 2 months of daily amikacin 150 mg/kg, ethionamide 50 mg/kg, moxifloxacin 100 mg/kg and pyrazinamide 150 mg/kg. Three negative control groups were treated for 2 months with one of the following drugs, rifampin 10 mg/kg daily, moxifloxacin 100 mg/kg daily and compound 12 25 mg/kg daily. All the tested regimens either for susceptible tuberculosis or for MDR tuberculosis are summarized in table 11. All the groups contained ten mice and were treated during 8 weeks from D14 to D70 five days a week. The parameters used for assessing the severity of infection and the effectiveness of treatments were : survival rate, spleen weight, gross lung lesions and CFU counts in the spleens and in the lungs.

Survival rate : The untreated mice began to die by day 21 after infection and all the mice were dead by day 28 of infection. All the treatments were able to prevent the mortality of mice and few mice died because of accident of gavage.

**Table 11. Experimental design**

| Dates of sacrifices | | | | Total mice | |
|---|---|---|---|---|---|
| | D-13 | D0 | 1month | 2 month | |
| Controls | | | | | |
| Untreated | 10 | 10 | 10 | | 30 |
| 2 Rifampicin | | | 10 | 10 | 20 |
| 2 Moxifloxacin | | | 10 | 10 | 20 |
| 2 compound 12 | | | 20* | 10 | 30 |
| Positive Controls | | | | | |
| 2 RMP+INH+PZA | | | 10 | 10 | 20 |
| 2 AMIK+ETHIO+MXFX+PZA | | | 10 | 10 | 20 |
| Tested regimens (Susceptible TB regimen) | | | | | |
| 2 RMP+INH | | | 10 | 10 | 20 |
| 2 RMP+ compound 12 | | | 10 | 10 | 20 |
| 2 INH+ compound 12 | | | 10 | 10 | 20 |
| 2 RMP+INH+ compound 12 | | | 10 | 10 | 20 |
| 2 INH+PZA+ compound 12 | | | 10 | 10 | 20 |
| 2 RMP+PZA+ compound 12 | | | 10 | 10 | 20 |
| 2 RMP+INH+PZA+ compound 12 | | | 10 | 10 | 20 |
| Tested regimens (Resistant TB regimen) | | | | | |
| 2AMIK+ETHIO+PZA | | | 10 | 10 | 20 |
| 2 AMIK+ETHIO +PZA+ compound 12 | | | 10 | 10 | 20 |
| 2 AMIK+MXFX+PZA | | | 10 | 10 | 20 |
| 2 AMIK +MXFX+PZA+ compound 12 | | | 10 | 10 | 20 |
| 2 AMIK+ETHIO+MXFX+PZA+compound 12 | | | 10 | 10 | 20 |
| Total | 10 | 10 | 190 | 170 | 380 |

| | | | | | |
|---|---|---|---|---|---|
| Dosages : Rifampicin (RMP)= 10 mg/kg, Isoniazid (INH) = 25 mg/kg, Pyrazinamide (PZA) =150 mg/kg, Amikacin (AMIK) = 150 mg/kg, Ethionamide (ETHIO) = 50 mg/kg, Moxifloxacin (MXFX) = 100 mg/kg, compound 12 = 25 mg/kg *: for serum dosage | | | | | |

The following Table shows the results of the 2 month experiment.

**Table 12. Mean spleen weight and number of CFU per spleen and lung of M.tuberculosis-infected mice and treated with various treatments for 2 months.**

| Group^{a} | No. mice | Spleen weight (mg) | Mean CFU (log₁₀) per | |
|---|---|---|---|---|
| | | | Spleen | Lung |
| Pretreatment | 10 | 631±121 | 6.40±0.30 | 6.94±0.51 |
| R 10 mg/kg | 9 | 391±70 | 2.75±0.34 | 1.89±0.50 |
| M 100 mg/kg | 10 | 400±99 | 3.53±0.34 | 2.89±0.57 |
| J 25 mg/kg | 8 | 248±47 | 1.24±0.50 | 0.22±0.32 |
| RHZ | 10 | 326±78 | 1.91±0.52 | 0.97±0.61 |
| AEtZM | 10 | 331±86 | 1.60±0.38 | 0.10±0.09 |
| RH | 10 | 400±100 | 2.49±0.42 | 1.09±0.36 |
| RJ | 9 | 304±61 | 2.06±0.61 | 1.63±0.77 |
| HJ | 8 | 293±56 | 1.27±0.31 | 0.36±0.40 |
| RHJ | 9 | 297±74 | 0.64±0.63 | 0.19±0.36 |
| HZJ | 7 | 257±40 | 0.07±0 | 0.07±0 |
| RZJ | 9 | 281±56 | 0.07±0 | 0.07±0 |
| RHZJ | 10 | 265±47 | 0.12±0.15 | 0.07±0 |
| AEtZ | 10 | 344±46 | 2.75±0.25 | 1.20±0.26 |
| AEtZJ | 9 | 331±86 | 0.10±0.10 | 0.07±0 |
| AMZ | 10 | 287±31 | 1.89±0.51 | 0.75±0.55 |
| AMZJ | 8 | 296±63 | 0.07±0 | 0.07±0 |
| AEtMZJ | 8 | 285±53 | 0.07±0 | 0.07±0 |

| | | | | |
|---|---|---|---|---|
| ^{a}: Except the pretreatment values were obtained from mice sacrificed on day 14 after inoculation, the remaining results were obtained from mice sacrificed on day 42 after inoculation. Treatment began on day 14, and was administered five time weekly for four weeks. Isoniazid (H), rifampin (R), moxifloxacin (M), pyrazinamide (Z), compound 12 (J), amikacin (A), ethionamide (Et). | | | | |

### In vitro testing of susceptibility to compound 12 of fully susceptible and multi drug resistant M.tuberculosis strains in solid medium assay.

The susceptibility to compound 12 of 73 *M. tuberculosis* strains was tested in a solid medium assay (agar plates). The panel of strains included strains (41) fully susceptible to standard anti-tuberculosis drugs as well as multi drug resistant (MDR) strains (32). i.e. strains resistant to at least rifampin and isoniazid.

Agar plates were welded with solutions containing compound 12 in a concentration ranging from 0.002 mg/L to 0.256 mg/L (8 different concentrations tested). *M. tuberculosis* isolates were then plated on each agar plate and the plates were sealed and incubated at 36°C for 3 weeks.

Isolate growth was analyzed 3 weeks following plate inoculation and an isolate's MIC was defined as the first concentration at which no growth was observed.

For all the tested strains, no growth was seen at concentrations higher than 0.064 mg/L, the majority of strains showed an MIC of 0.032 mg/L.

No difference in MIC was seen between fully susceptible and MDR *M. tuberculosis* strains.

### In vivo testing of susceptibility of M tuberculosis to compound 12 in combination with other antimycobacterial agents.

Swiss mice were inoculated intravenously with 10⁶ log colony forming units (CFU) of strain H37Rv. Compound 12 (J) was administrated by gavage 5 days/week (once a day treatment group) or once a week from day 14 to day 70 after inoculation, in monotherapy or in association with isoniazid (H), rifampin (R), pyrazinamide (Z), or moxifloxacin (M). The lung CFU was determined after I or 2 months of treatment. The results are gathered in Tables 13 and 14.

**Table 13 : Results for once-a-day group after 1 and 2 months**

| | CFU | | % positive mice | Decrease 1mo | Decrease 2mo |
|---|---|---|---|---|---|
| | 1 month | 2 months | 2nd month | vs D0 | vs D0 |
| D0 | 7.23 | | | | |
| R | 6.01 | 4.07 | 10/10 | -1.22 | -3.16 |
| H | 4.89 | 4.72 | 10/10 | -2.34 | -2.51 |
| Z | 6.17 | 6.43 | 7/7 | -1.06 | -0.8 |
| M | 5.51 | 4.3 | 10/10 | -1.72 | -2.93 |
| J | 4.14 | 2.28 | 8/10 | -3.09 | -4.95 |
| RH | 5.07 | 3.12 | 10/10 | -2.16 | -4.11 |
| RZ | 5.38 | 1.91 | 8/10 | -1.85 | -5.32 |
| HZ | 5.47 | 3.93 | 10/10 | -1.76 | -3.3 |
| RM | 5.52 | 3.13 | 8/10 | -1.71 | -4.1 |
| JR | 4.67 | 1.89 | 7/10 | -2.56 | -5.34 |
| JH | 3.75 | 1.91 | 8/10 | -3.48 | -5.32 |
| JZ | 1.61 | 0 | 0/10 | -5.62 | -7.23 |
| JM | 4.61 | 2.13 | 7/9 | -2.62 | -5.1 |
| RHZ | 3.87 | 2.22 | 9/9 | -3.36 | -5.01 |
| RMZ | 4.59 | 1.36 | 8/10 | -2.64 | -5.87 |
| JHZ | 1.71 | 0.18 | 2/9 | -5.52 | -7.05 |
| JHR | 4.37 | 1.15 | 8/10 | -2.86 | -6.08 |
| JMR | 4.42 | 1.37 | 8/9 | -2.81 | -5.86 |
| JRZ | 2.31 | 0.07 | 3/10 | -4.92 | -7.16 |
| JMZ | 1.44 | 0.03 | 2/9 | -5.79 | -7.2 |

**Table 14 : Results for once-a-week group after 2 months**

| | lung CFU* | % positive mice |
|---|---|---|
| D0 | 7.23 | |
| J | 1,99+/-0,75 | 9/9 |
| M | 6,44+/-0,5 | 7/7 |
| P | 3,26+/-0,58 | 10/10 |
| JP | 1,63+/-0,92 | 8/9 |
| JPM | 1,85+/-0,7 | 10/10 |
| JPH | 1,48+/-0,79 | 10/10 |
| JPZ | 0,23+/-0,72 | 1/10 |

## Claims

1. Use of a substituted quinoline derivative for the preparation of a medicament for the treatment of an infection with a drug resistant Mycobacterium strain wherein the substituted quinoline derivative is a compound according to Formula (Ia) or Formula (Ib) 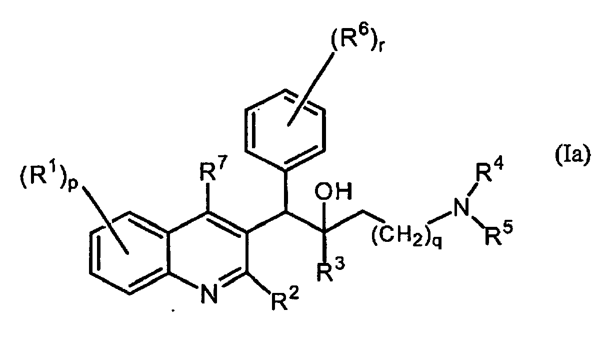 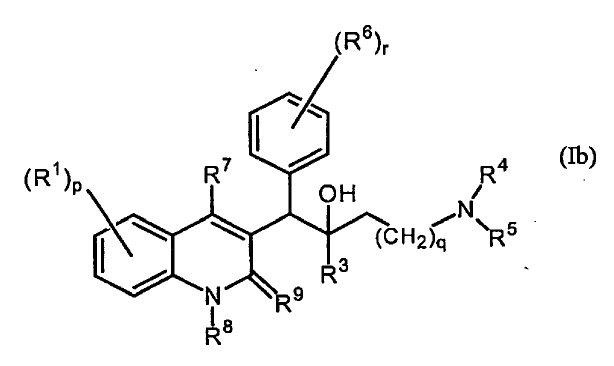 a pharmaceutically acceptable acid or base addition salt thereof, a stereochemically isomeric form thereof, a tautomeric form thereof or a *N*-oxide form thereof, wherein:
R¹ is hydrogen, halo, haloalkyl, cyano, hydroxy, Ar, Het, alkyl, alkyloxy, alkylthio, alkyloxyalkyl, alkylthioalkyl, Ar-alkyl or di(Ar)alkyl ;
p is an integer equal to 1, 2, 3 or 4 ;
R² is hydrogen, hydroxy, mercapto, alkyloxy, alkyloxyalkyloxy, alkylthio, mono or di(alkyl)amino or a radical of formula 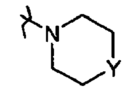 wherein Y is CH₂, O, S, NH or N-alkyl ;
R³ is alkyl, Ar, Ar-alkyl, Het or Het-alkyl;
q is an integer equal to zero, 1,2, 3 or 4 ;
R⁴ and R⁵ each independently are hydrogen, alkyl or benzyl; or
R⁴ and R⁵ together and including the N to which they are attached may form a radical selected from the group of pyrrolidinyl, 2*H*-pyrrolyl, 2-pyrrolinyl, 3-pyrrolinyl, pyrrolyl, imidazolidinyl, pyrazolidinyl, 2-imidazolinyl, 2-pyrazolinyl, imidazolyl, pyrazolyl, triazolyl, piperidinyl, pyridinyl, piperazinyl, imidazolidinyl, pyridazinyl, pyrimidinyl, pyrazinyl, triazinyl, morpholinyl and thiomorpholinyl, optionally substituted with alkyl, halo, haloalkyl, hydroxy, alkyloxy, amino, mono- or dialkylamino, alkylthio, alkyloxyalkyl, alkylthioalkyl and pyrimidinyl ;
R⁶ is hydrogen, halo, haloalkyl, hydroxy, Ar, alkyl, alkyloxy, alkylthio, alkyloxyalkyl, alkylthioalkyl, Ar-alkyl or di(Ar)alkyl ; or
two vicinal R⁶ radicals may be taken together to form a bivalent radical of formula -CH=CH-CH=CH- ;
r is an integer equal to 1,2,3,4 or 5 ; and
R⁷ is hydrogen, alkyl, Ar or Het;
R⁸ is hydrogen or alkyl ;
R⁹ is oxo ; or
R⁸ and R⁹ together form the radical =N-CH=CH-;
alkyl is a straight or branched saturated hydrocarbon radical having from 1 to 6 carbon atoms ; or is a cyclic saturated hydrocarbon radical having from 3 to 6 carbon atoms ; or is a a cyclic saturated hydrocarbon radical having from 3 to 6 carbon atoms attached to a straight or branched saturated hydrocarbon radical having from 1 to 6 carbon atoms ; wherein each carbon atom can be optionally substituted with halo, hydroxy, alkyloxy or oxo ;
Ar is a homocycle selected from the group of phenyl, naphthyl, acenaphthyl, tetrahydronaphthyl, each optionally substituted with 1, 2 or 3 substituents, each substituent independently selected from the group of hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl and mono- or dialkylaminocarbonyl ;
Het is a monocyclic heterocycle selected from the group of *N*-phenoxypiperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl and pyridazinyl; or a bicyclic heterocycle selected from the group of quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl or benzo[1,3]dioxolyl ; each monocyclic and bicyclic heterocycle may optionally be substituted on a carbon atom with 1, 2 or 3 substituents selected from the group of halo, hydroxy, alkyl or alkyloxy;
halo is a substituent selected from the group of fluoro, chloro, bromo and iodo and
haloalkyl is a straight or branched saturated hydrocarbon radical having from 1 to 6 carbon atoms or a cyclic saturated hydrocarbon radical having from 3 to 6 carbon atoms, wherein one or more carbon atoms are substituted with one or more halo-atoms.

2. Use according to claim 1 wherein R⁶ in Formula (Ia) or (Ib) is hydrogen, halo, haloalkyl, hydroxy, Ar, alkyl, alkyloxy, alkylthio, alkyloxyalkyl, alkylthioalkyl, Ar-alkyl or di(Ar)alkyl.

3. Use according to claim 1 or 2 wherein in Formula (Ia) or (Ib) R¹ is halo.

4. Use according to claim 3 wherein in Formula (Ia) or (Ib) R¹ is bromo.

5. Use according to any one of the preceding claims wherein in Formula (Ia) or (Ib) p is equal to 1.

6. Use according to any one of the preceding claims wherein in Formula (Ia) or (Ib) R² is alkyloxy.

7. Use according to claim 6 wherein in Formula (Ia) or (Ib) R² is C₁₋₄alkyloxy.

8. Use according to claim 7 wherein in Formula (Ia) or (Ib) R² is methyloxy.

9. Use according to any one of the preceding claims wherein in Formula (Ia) or (Ib) R³ is naphthyl or phenyl, each optionally substituted with halo.

10. Use according to claim 9 wherein R³ is naphthyl.

11. Use according to any one of the preceding claims wherein in Formula (Ia) or (Ib) q is equal to 1.

12. Use according to any one of the preceding claims wherein in Formula (Ia) or (Ib) R⁴ and R⁵ each independently are hydrogen or alkyl.

13. Use according to claim 12 wherein R⁴ and R⁵ each independently are C₁₋₄alkyl.

14. Use according to claim 13 wherein R⁴ and R⁵ each independently are methyl.

15. Use according to any one of the preceding claims wherein in Formula (Ia) or (Ib) R⁶ is hydrogen.

16. Use according to any one of the preceding claims wherein in Formula (Ia) or (Ib) R⁷ is hydrogen.

17. Use according to any one of the preceding claims wherein in Formula (Ia) or (Ib) haloalkyl is trifluoromethyl.

18. Use according to any one of the preceding claims wherein in Formula (Ia) or (Ib) alkyl is methyl or ethyl.

19. Use according to any one of the preceding claims wherein the substituted quinoline derivative is a compound according to Formula (Ia).

20. Use according to claim 1 wherein the substituted quinoline derivative is a compound according to Formula (Ia) in which R¹ is hydrogen, halo, Ar, alkyl or alkyloxy, p = 1, R² is hydrogen, alkyloxy or alkylthio, R³ is naphthyl, phenyl or thienyl, each optionally substituted with 1 or 2 substituents selected from the group of halo and haloalkyl, q = 0, 1, 2 or 3, R⁴ and R⁵ each independently are hydrogen or alkyl or R⁴ and R⁵ together and including the N to which they are attached form a radical selected from the group of imidazolyl, triazolyl, piperidinyl, piperazinyl and thiomorpholinyl, R⁶ is hydrogen, alkyl or halo, r is equal to 0 or 1 and R⁷ is hydrogen.

21. Use according to claim 1, **characterized in that** the compound is selected from the group consisting of :
o 1-(6-bromo-2-methoxy-quinolin-3-yl)-2-(3,5-difluoro-phenyl)-4-dimethylamino-1-phenyl-butan-2-ol ;
o 1-(6-bromo-2-mcthoxy-quinolin-3-yl)-4-dimethylamino-2-naphthalen-1-yl-1-phenyl-butan-2-ol ;
o 1-(6-bromo-2-methoxy-quinolin-3-yl)-2-(2,5-difluoro-phenyl)-4-dimethylamino-1-phenyl-butan-2-ol ;
o 1-(6-bromo-2-methoxy-quinolin-3-yl)-2-(2,3-difluoro-phenyl)-4-dimethylamino-1-phenyl-butan-2-ol ;
o 1-(b-bromo-2-methoxy-quinolin-3-yl)-4-dimethylamino-2-(2-fluoro-phenyl)-1-phenyl-butan-2-ol ;
o 1-(6-bromo-2-methoxy-quinolin-3-yl)-4-dimethylamino-2-naphthalen-1-yl-1-p-tolyl-butan-2-ol ;
o 1-(6-bromo-2-methoxy-quinolin-3-yl)-4-methylamino-2-naphthalen-1-yl-1-phenyl-butan-2-ol ;
o 1-(6-bromo-2-methoxy-quinolin-3-yl)-4-dimethylamino-2-(3-fluoro-phenyl)-1-phenyl-butan-2-ol; and
o 1-(6-bromo-2-methoxy-quinolin-3-yl)-4-dimethylamino-2-phenyl-l-phenyl-butan-2-ol;
a pharmaceutically acceptable acid or base addition salt thereof, a stereochemically isomeric form thereof or a *N*-oxide form thereof.

22. Use according to claim 21 wherein the compound is selected from the group consisting of
o 1-(6-bromo-2-methoxy-quinolin-3-yl)-4-dimethylamino-2-(3-fluoro-phenyl)-1-phenyl-butan-2-ol;
o 1-(6-bromo-2-methoxy-quinolin-3-yl)-4-dimethylamino-2-phenyl-1-phenyl-butan-2-ol;
o 1-(6-bromo-2-methoxy-quinolin-3-yl)-4-dimethylamino-2-naphthalen-1-yl-1-phenyl-butan-2-ol ;
a pharmaceutically acceptable acid or base addition salt thereof, a stereochemically isomeric form thereof or a *N*-oxide form thereof.

23. Use according to claim 1 wherein the compound is 6-bromo-α-[2-(dimethylamino)ethyl]-2-methoxy-α-1-naphthalenyl-β-phenyl-3-quinolineethanol, a pharmaceutically acceptable acid or base addition salt thereof, a stereochemically isomeric form thereof or a *N*-oxide form thereof.

24. Use according to claim 23 wherein the compound is 6-bromo-α-[2-(dimethylamino)ethyl]-2-methoxy-α-1-naphthalenyl-β-phenyl-3-quinolineethanol, or a pharmaceutically acceptable acid addition salt thereof.

25. Use according to claim 23 wherein the compound is 6-bromo-α-[2-(dimethylamino)ethyl]-2-methoxy-α-1-naphthalenyl-β-phenyl-3-quinolineethanol, or a stereochemically isomeric form thereof.

26. Use according to claim 23 wherein the compound is 6-bromo-α-[2-(dimethylamino)ethyl]-2-methoxy-α-1-naphthalenyl-β-phenyl-3-quinolineethanol, or a *N*-oxide form thereof.

27. Use according to claim 23 wherein the compound is (αS, βR)-6-bromo-α-[2-(dimethylamino)ethyl]-2-methoxy-α-1-naphthalenyl-β-phenyl-3-quinolineethanol, or a pharmaceutically acceptable acid addition salt thereof.

28. Use according to claim 27 wherein the compound is (αS, βR)-6-bromo-α-[2-(dimcthylamino)ethyl]-2-methoxy-α-1-naphthalenyl-β-phenyl-3-quinolineethanol.

29. Use of a substituted quinoline derivative for the preparation of a medicament for the treatment of an infection with a drug resistant Mycobacterium strain wherein the substituted quinoline derivative has the following structure 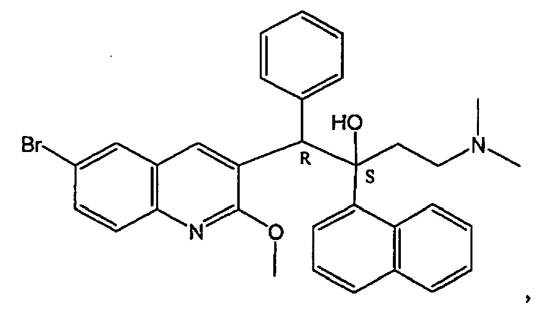 or a pharmaceutically acceptable acid addition salt thereof.

30. Use according to any one of the preceding claim wherein the drug resistant Mycobacterium strain is multi drug resistant.

31. Use according to any one of the preceding claims wherein the Mycobacterium strain is a Mycobacterium tuberculosis strain.

32. A combination of (a) a compound of formula (Ia) or (Ib) as defined in any one of claims 1 to 29 and (b) one or more other antimycobacterial agents.

33. A combination of (a) a compound of formula (Ia) or (Ib) as defined in any one of claims 1 to 29 and (b) one or more other antimycobacterial agents for use as a medicine.

34. A pharmaceutical composition comprising a pharmaceutically acceptable carrier and, as active ingredient, a therapeutically effective amount of (a) a compound of formula (Ia) or (Ib) as defined in any one of claims 1 to 29 and (b) one or more other antimycobacterial agents.

35. A product containing (a) a compound of formula (Ia) or (Ib) as defined in any one of claims 1 to 29, and (b) one or more other antimycobacterial agents, as a combined preparation for simultaneous, separate or sequential use in the treatment of mycobacterial diseases.

36. A combination, a pharmaceutical composition or a product as claimed in any one of claims 32 to 35 wherein the one or more other antimycobacterial agents comprise pyrazinamide.

37. A combination, a pharmaceutical composition or a product as claimed in any one of claims 32 to 35 wherein the one or more other antimycobacterial agents are selected from rifampicin (=rifampin); isoniazid; pyrazinamide; amikacin; ethionamide; moxifloxacin; ethambutol; streptomycin; para-aminosalicylic acid; cycloserine; capreomycin; kanamycin; thioacetazone; PA-824; quinolones/fluoroquinolones such as for example ofloxacin, ciprofloxacin, sparfloxacin; macrolides such as for example clarithromycin, clofazimine, amoxycillin with clavulanic acid; rifamycins; rifabutin; rifapentine.

38. A combination, a pharmaceutical composition or a product as claimed in any one of claims 32 to 37 wherein the compound of formula (Ia) or (Ib) is (αS, βR)-6-bromo-α-[2-(dimethylamino)ethyl]-2-methoxy-α-1-naphthalenyl-β-phenyl-3-quinolineethanol or a pharmaceutically acceptable acid addition salt thereof.

39. A combination, a pharmaceutical composition or a product as claimed in any one of claims 32 to 38 wherein the compound of formula (Ia) or (Ib) is (αS, βR)-6-bromo-α-[2-(dimethylamino)ethyl]-2-methoxy-α-1-naphthalenyl-β-phenyl-3-quinolineethanol.

40. Use of a combination, a pharmaceutical composition or a product as claimed in any one of claims 32 to 39 for the preparation of a medicament for the treatment of an infection with a drug resistant Mycobacterium strain.

41. Use according to claim 40 wherein the drug resistant Mycobacterium strain is a drug resistant *Mycobacterium tuberculosis* strain.

## Patentansprüche

1. Verwendung eines substituierten Chinolinderivats für die Herstellung eines Arzneimittels zur Behandlung einer Infektion mit einem arzneimittelresistenten Mycobakterienstamm, wobei es sich bei dem substituierten Chinolinderivat um eine Verbindung gemäß Formel (Ia) oder Formel (Ib) 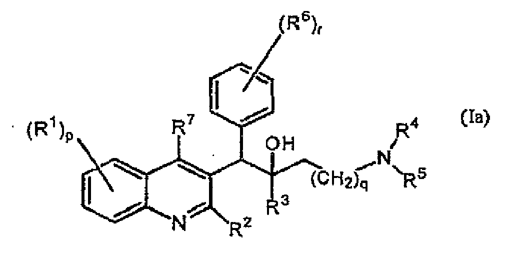 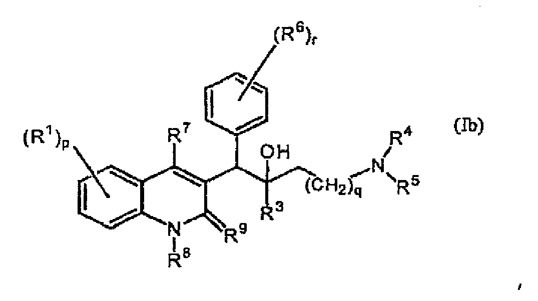 ein pharmazeutisch annehmbares Säure- oder Basenadditionssalz davon, eine stereochemisch isomere Form davon, eine tautomere Form davon oder eine *N-*Oxidform davon handelt, mit den folgenden Bedeutungen:
R¹ bedeutet Wasserstoff, Halogen, Halogenalkyl, Cyano, Hydroxy, Ar, Het, Alkyl, Alkyloxy, Alkylthio, Alkyloxyalkyl, Alkylthioalkyl, Ar-Alkyl oder Di(Ar)alkyl;
p bedeutet eine ganze Zahl gleich 1, 2, 3 oder 4;
R² bedeutet Wasserstoff, Hydroxy, Mercapto, Alkyloxy, Alkyloxyalkyloxy, Alkylthio, Mono- oder Di(alkyl)amino oder einen Rest der Formel 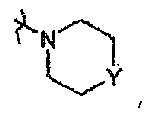 wobei Y CH₂, O, S, NH oder N-Alkyl bedeutet;
R³ bedeutet Alkyl, Ar, Ar-Alkyl, Het oder Het-Alkyl;
q bedeutet eine ganze Zahl gleich Null, 1, 2, 3 oder 4;
R⁴ und R⁵ bedeuten jeweils unabhängig voneinander Wasserstoff, Alkyl oder Benzyl oder
R⁴ und R⁵ gemeinsam und inklusive dem N, an den sie gebunden sind, können einen Rest aus der Gruppe Pyrrolidinyl, 2H-Pyrrolyl, 2-Pyrrolinyl, 3-Pyrrolinyl, Pyrrolyl, Imidazolidinyl, Pyrazolidinyl, 2-Imidazolinyl, 2-Pyrazolinyl, Imidazolyl, Pyrazolyl, Triazolyl, Piperidinyl, Pyridinyl, Piperazinyl, Imidazolidinyl, Pyridazinyl, Pyrimidinyl, Pyrazinyl, Triazinyl, Morpholinyl und Thiomorpholinyl, die gegebenenfalls durch Alkyl, Halogen, Halogenalkyl, Hydroxy, Alkyloxy, Amino, Mono- oder Dialkylamino, Alkylthio, Alkyloxyalkyl, Alkthylthioalkyl und Pyrimidinyl substituiert sind, bilden;
R⁶ bedeutet Wasserstoff, Halogen, Halogenalkyl, Hydroxy, Ar, Alkyl, Alkyloxy, Alkylthio, Alkyloxyalkyl, Alkylthioalkyl, Ar-Alkyl oder Di(Ar)alkyl oder
zwei benachbarte R⁶-Reste können gemeinsam einen zweiwertigen Rest der Formel -CH=CH-CH=CH- bilden;
r bedeutet eine ganze Zahl gleich 1, 2, 3, 4 oder 5 und
R⁷ bedeutet Wasserstoff, Alkyl, Ar oder Het;
R⁸ bedeutet Wasserstoff oder Alkyl;
R⁹ bedeutet Oxo oder
R⁸ und R⁹ gemeinsam bilden den Rest =N-CH=CH-;
Alkyl bedeutet einen geradkettigen oder verzweigten gesättigten Kohlenwasserstoffrest mit 1 bis 6 Kohlenstoffatomen, oder einen cyclischen gesättigten Kohlenwasserstoffrest mit 3 bis 6 Kohlenstoffatomen, oder einen cyclischen gesättigten Kohlenwasserstoffrest mit 3 bis 6 Kohlenstoffatomen, der an einen geradkettigen oder verzweigten gesättigten Kohlenwasserstoffrest mit 1 bis 6 Kohlenstoffatomen gebunden ist; wobei jedes Kohlenstoffatom gegebenenfalls durch Halogen, Hydroxy, Alkyloxy oder Oxo substituiert sein kann;
Ar bedeutet einen Homocyclus aus der Gruppe Phenyl, Naphtyl, Acenaphtyl, Tetrahydronaphtyl, die jeweils gegebenenfalls durch 1, 2 oder 3 Substituenten substituiert sind, wobei jeder Substituent unabhängig aus der Gruppe Hydroxy, Halogen, Cyano, Nitro, Amino, Mono- oder Dialkylamino, Alkyl, Halogenalkyl, Alkyloxy, Halogenalkyloxy, Carboxyl, Alkyloxycarbonyl, Aminocarbonyl, Morpholinyl und Mono- oder Dialkylaminocarbonyl stammt;
Het beudetet einen monocyclischen Heterocyclus aus der Gruppe *N-*Phenoxypiperidinyl, Pyrrolyl, Pyrazolyl, Imidazolyl, Furanyl, Thienyl, Oxazolyl, Isoxazolyl, Thiazolyl, Isothiazolyl, Pyridinyl, Pyrimidinyl, Pyrazinyl und Pyridazinyl, oder einen bicyclischen Heterocyclus aus der Gruppe Chinolinyl, Chinoxalinyl, Indolyl, Benzimidazolyl, Benzoxazolyl, Benzisoxazolyl, Benzothiazolyl, Benzisothiazolyl, Benzofuranyl, Benzothienyl, 2,3-Dihydrobenzo[1,4]dioxinyl oder Benzo[1,3]dioxolyl wobei jeder monocyclische und bicyclische Heterocyclus gegebenenfalls an einem Kohlenstoffatom durch 1, 2 oder 3 Substituenten aus der Gruppe Halogen, Hydroxy, Alkyl oder Alkyloxy substituiert sein kann;
Halogen bedeutet einen Substituenten aus der Gruppe Fluor, Chlor, Brom und Iod und
Halogenalkyl bedeutet einen geradkettigen oder verzweigten gesättigten Kohlenwasserstoffrest mit 1 bis 6 Kohlenstoffatomen oder einen cyclischen gesättigten Kohlenwasserstoffrest mit 3 bis 6 Kohlenstoffatomen, wobei eines oder mehrere Kohlenstoffatome durch ein oder mehrere Halogenatome substituiert sind.

2. Verwendung nach Anspruch 1, wobei R⁶ in Formel (Ia) oder (Ib) Wasserstoff, Halogen, Halogenalkyl, Hydroxy, Ar, Alkyl, Alkyloxy, Alkylthio, Alkyloxyalkyl, Alkylthioalkyl, Ar-Alkyl oder Di(Ar)alkyl bedeutet.

3. Verwendung nach Anspruch 1 oder 2, wobei R¹ in Formel (Ia) oder (Ib) Halogen bedeutet.

4. Verwendung nach Anspruch 3, wobei R¹ in Formel (Ia) oder (Ib) Brom bedeutet.

5. Verwendung nach einem der vorhergehenden Ansprüche, wobei p in Formel (Ia) oder (Ib) gleich 1 ist.

6. Verwendung nach einem der vorhergehenden Ansprüche, wobei R² in Formel (Ia) oder (Ib) Alkyloxy bedeutet.

7. Verwendung nach Anspruch 6, wobei R² in Formel (Ia) oder (Ib) C₁₋₄-Alkyloxy bedeutet.

8. Verwendung nach Anspruch 7, wobei R² in Formel (Ia) oder (Ib) Methyloxy bedeutet.

9. Verwendung nach einem der vorhergehenden Ansprüche wobei R³ in Formel (Ia) oder (Ib) Naphtyl oder Phenyl, die jeweils gegebenenfalls durch Halogen substituiert sind, bedeutet.

10. Verwendung nach Anspruch 9, wobei R³ Naphtyl bedeutet.

11. Verwendung nach einem der vorhergehenden Ansprüche, wobei q in Formel (Ia) oder (Ib) gleich 1 ist.

12. Verwendung nach einem der vorhergehenden Ansprüche, wobei R⁴ und R⁵ in Formel (Ia) oder (Ib) jeweils unabhängig Wasserstoff oder Alkyl bedeuten.

13. Verwendung nach Anspruch 12, wobei R⁴ und R⁵ jeweils unabhängig C₁₋₄-Alkyl bedeuten.

14. Verwendung nach Anspruch 13, wobei R⁴ und R⁵ jeweils unabhängig Methyl bedeuten.

15. Verwendung nach einem der vorhergehenden Ansprüche, wobei R⁶ in Formel (Ia) oder (Ib) Wasserstoff bedeutet.

16. Verwendung nach einem der vorhergehenden Ansprüche, wobei R⁷ in Formel (Ia) oder (Ib) Wasserstoff bedeutet.

17. Verwendung nach einem der vorhergehenden Ansprüche, wobei Halogenoalkyl in Formel (Ia) oder (Ib) Trifluormethyl bedeutet.

18. Verwendung nach einem der vorhergehenden Ansprüche, wobei Alkyl in Formel. (Ia) oder (Ib) Methyl oder Ethyl bedeutet.

19. Verwendung nach einem der vorhergehenden Ansprüche, wobei es sich bei dem substituierten Chinolinderivat um eine Verbindung gemäß Formel (Ia) handelt.

20. Verwendung nach Anspruch 1, wobei es sich bei dem substituierten Chinolinderivat um eine Verbindung gemäß Formel (Ia) handelt, wobei R¹ Wasserstoff, Halogen, Ar, Alkyl oder Alkyloxy bedeutet, p gleich 1 ist, R² Wasserstoff, Alkyloxy oder Alkylthio bedeutet, R³ Naphtyl, Phenyl oder Thienyl, die jeweils gegebenenfalls durch 1 oder 2 Substituenten aus der Gruppe Halogen und Halogenalkyl substituiert sind, bedeutet, q gleich 0, 1, 2 oder 3 ist, R⁴ und R⁵ jeweils unabhängig Wasserstoff oder Alkyl bedeuten oder R⁴ und R⁵ gemeinsam und inklusive dem N, an den sie gebunden sind, einen Rest aus der Gruppe Imidazolyl, Triazolyl, Piperidinyl, Piperazinyl und Thiomorpholinyl bilden, R⁶ Wasserstoff, Alkyl oder Halogen bedeutet, r gleich 0 oder 1 ist und R⁷ Wasserstoff bedeutet.

21. Verwendung nach Anspruch 1, **dadurch gekennzeichnet, daß** die Verbindung aus der folgenden Gruppe stammt:
o 1-(6-Brom-2-methoxychinolin-3-yl)-2-(3,5-difluorphenyl)-4-dimethylamino-1-phenylbutan-2-ol;
o 1-(6-Brom-2-methoxychinolin-3-yl)-4-dimethylamino-2-naphtalin-1-yl-1-phenylbutan-2-ol;
o 1-(6-Brom-2-methoxychinolin-3-yl)-2-(2,5-difluorphenyl)-4-dimethylamino-1-phenylbutan-2-ol;
o 1-(6-Brom-2-methoxychinolin-3-yl)-2-(2,3-difluorphenyl)-4-dimethylamino-1-phenylbutan-2-ol;
o 1-(6-Brom-2-methoxychinolin-3-yl)-4-dimethylamino-2-(2-f1uorphenyl)-1-phenylbutan-2-ol;
o 1-(6-Brom-2-methoxychinolin-3-yl)-4-dimethylamino-2-naphtalin-1-yl-1-p-tolylbutan-2-ol;
o 1-(6-Brom-2-methoxychinolin-3-y1)-4-methylamino-2-naphtalin-yl-1-phenylbutan-2-ol;
o 1-(6-Brom-2-methoxychinolin-3-yl)-4-dimethylamino-2-(3-fluorphenyl)-1-phenylbutan-2-ol und
o 1-(6-Brom-2-methoxychinolin-3-yl)-4-dimethylamino-2-phenyl-1-phenylbutan-2-ol;
einem pharmazeutisch annehmbaren Säure- oder Basenadditionssalz davon, einer stereochemisch isomeren Form davon, oder einer *N-*Oxidform davon.

22. Verwendung nach Anspruch 21, **dadurch gekennzeichnet, daß** die Verbindung aus der folgenden Gruppe stammt:
o 1-(6-Brom-2-methoxychinolin-3-yl)-4-dimethylamino-2-(3-fluorphenyl)-1-phenylbutan-2-o1;
o 1-(6-Brom-2-methoxychinolin-3-yl)-4-dimethylamino-2-phenyl-1-phenylbutan-2-ol;
o 1-(6-Brom-2-methoxychinolin-3-yl)-4-dimethylamzno-2-naphtalin-1-yl-1-phenylbutan-2-ol;
einem pharmazeutisch annehmbaren Säure- oder Basenadditionssalz davon, einer stereochemisch isomeren Form davon, oder einer *N-*Oxidform davon.

23. Verwendung nach Anspruch 1, wobei es sich bei der Verbindung um 6-Brom-α-[2-(dimethylamino)ethyl]-2-methoxy-α-1-naphtalinyl-β-phenyl-3-chinolinethanol, ein pharmazeutisch annehmbares Säure- oder Basenadditionssalz davon, eine stereochemisch isomere Form davon, oder eine *N-*Oxidform davon handelt.

24. Verwendung nach Anspruch 23, wobei es sich bei der Verbindung um 6-Brom-α-[2-(dimethylamino)ethyl]-2-methoxy-α-1-naphtalinyl-β-phenyl-3-chinolinethanol oder ein pharmazeutisch annehmbares Säureadditionssalz davon handelt.

25. Verwendung nach Anspruch 23, wobei es sich bei der Verbindung um 6-Brom-α-[2-(dimethylamino)ethyl]-2-methoxy-α-1-naphtalinyl-β-phenyl-3-chinolinethanol oder eine stereochemisch isomere Form davon handelt.

26. Verwendung nach Anspruch 23, wobei es sich bei der Verbindung um 6-Brom-α-[2-(dimethylamino)ethyl]-2-methoxy-α-1-naphtalinyl-β-phenyl-3-chinolinethanol oder eine *N-*Oxidform davon handelt.

27. Verwendung nach Anspruch 23, wobei es sich bei der Verbindung um (αS,βR)-6-Brom-α-[2-(dimethylamino)ethyl]-2-methoxy-α-1-naphtalinyl-β-phenyl-3-chinolinethanol oder ein pharmazeutisch annehmbares Säureadditionssalz davon handelt.

28. Verwendung nach Anspruch 27, wobei es sich bei der Verbindung um (αS,βR)-6-Brom-α-[2-(dimethylamino)ethyl]-2-methoxy-α-1-naphtalinyl-β-phenyl-3-chinolinethanol handelt.

29. Verwendung eines substituierten Chinolinderivats für die Herstellung eines Arzneimittels zur Behandlung einer Infektion mit einem arzneimittelresistenten Mycobakterienstamm, wobei das susbtituierte Chinolinderivat die folgende Struktur aufweist 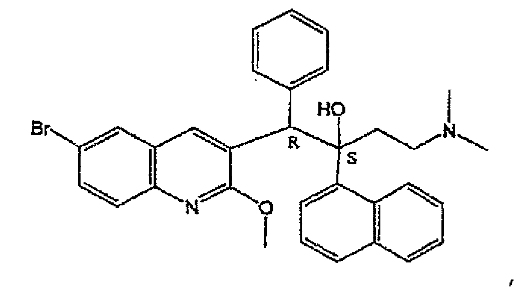 oder ein pharmazeutisch annehmbares Säureadditionssalz davon.

30. Verwendung nach einem der vorhergehenden Ansprüche, wobei der arzneimittelresistente Mycobakterienstamm gegen mehrere Arzneistoffe resistent ist.

31. Verwendung nach einem der vorhergehenden Ansprüche, wobei es sich bei dem Mycobakterienstamm um einen Stamm von *Mycobacterium tuberculosis* handelt.

32. Kombination von (a) einer Verbindung der Formel (Ia) oder (Ib) wie in einem der Ansprüche 1 bis 29 definiert und (b) einem oder mehreren sonstigen antimycobakteriellen Mitteln.

33. Kombination von (a) einer Verbindung der Formel (Ia) oder (Ib) wie in einem der Ansprüche 1 bis 29 definiert und (b) einem oder mehreren sonstigen antimycobakteriellen Mitteln zur Verwendung als Arzneimittel.

34. Pharmazeutische Zusammensetzung, umfassend einen pharmazeutisch annehmbaren Träger und als Wirkstoff eine therapeutisch wirksame Menge von (a) einer Verbindung der Formel (Ia) oder (Ib) wie in einem der Ansprüche 1 bis 29 definiert und (b) einem oder mehreren sonstigen antimycobakteriellen Mitteln.

35. Produkt enthaltend (a) eine Verbindung der Formel (Ia) oder (Ib) wie in einem der Ansprüche 1 bis 29 definiert und (b) ein oder mehrere sonstigen antimycobakterielle Mitteln als Kombinationspräparat für die gleichzeitige, getrennte oder aufeinanderfolgende Verwendung bei der Behandlung von mycobakteriellen Krankheiten.

36. Kombination, pharmazeutische Zusammensetzung oder Produkt nach einem der Ansprüche 32 bis 35, wobei das eine oder die mehreren sonstigen antimycobakteriellen Mittel Pyrazinamid umfaßt bzw. umfassen.

37. Kombination, pharmazeutische Zusammensetzung oder Produkt nach einem der Ansprüche 32 bis 35, wobei das eine oder die mehreren sonstigen antimycobakteriellen Mittel aus der Reihe Rifampicin (=Rifampin); Isoniazid; Pyrazinamid; Amikacin; Ethionamid; Moxifloxacin; Ethambutol; Streptomycin; para-Aminosalicylsäure; Cycloserin; Capreomycin; Kanamycin; Thioacetazon; PA-824; Chinolone/Fluorchinolone wie zum Beispiel Ofloxacin, Ciprofloxacin, Sparfloxacin; Macrolide wie zum Beispiel Clarithromycin, Clofazimin, Amoxycillin mit Clavulansäure; Rifamycin; Rifabutin; Rifapentin stammen.

38. Kombination, pharmazeutische Zusammensetzung oder Produkt nach einem der Ansprüche 32 bis 37, wobei es sich bei der Verbindung der Formel (Ia) oder (Ib) um (αS,βR)-6-Brom-α-[2-(dimethylamino)ethyl]-2-methoxy-α-1-naphtalinyl-β-phenyl-3-chinolinethanol oder ein pharmazeutisch annehmbares Säureadditionssalz davon handelt.

39. Kombination, pharmazeutische Zusammensetzung oder Produkt nach einem der Ansprüche 32 bis 38, wobei es sich bei der Verbindung der Formel (Ia) oder (Ib) um (αS,βR)-6-Brom-α-[2-(dimethylamino)ethyl]-2-methoxy-α-1-naphtalinyl-β-phenyl-3-chinolinethanol handelt.

40. Verwendung einer Kombination, einer pharmazeutischen Zusammensetzung oder eines Produkts nach einem der Ansprüche 32 bis 39 für die Herstellung eines Arzneimittels für die Behandlung einer Infektion mit einem arzneimittelresistenten Mycobakterienstamm.

41. Verwendung nach Anspruch 40, wobei es sich bei dem arzneimittelresistenten Mycobakterienstamm um einen arzneimittelresistenten Stamm von *Mycobacterium tuberculosis* handelt.

## Revendications

1. Utilisation d'un dérivé de quinoléine substitué pour la préparation d'un médicament destiné au traitement d'une infection par une souche de *Mycobacterium* résistant aux médicaments, dans laquelle le dérivé de quinoléine substitué est un composé selon la formule (Ia) ou la formule (Ib) 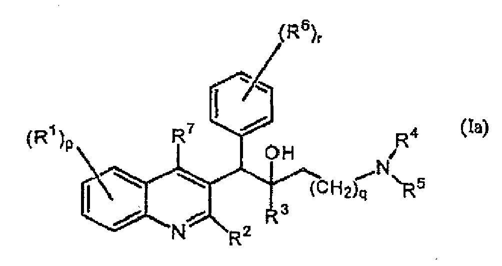 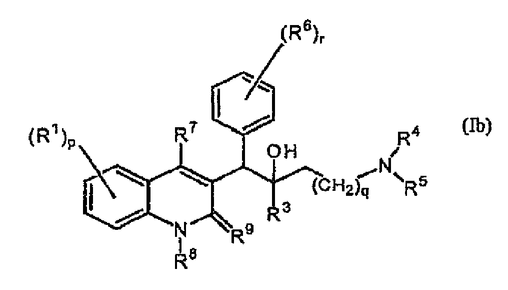 un sel pharmaceutiquement acceptable d'addition à un acide ou à une base de celui-ci, une forme stéréochimiquement isomère de celui-ci, une forme tautomère de celui-ci ou une forme *N*-oxyde de celui-ci,
dans lesquelles :
R¹ est un atome d'hydrogène, un groupe halogéno, un groupe halogénoalkyle, un groupe cyano, un groupe hydroxy, un groupe Ar, un groupe Het, un groupe alkyle, un groupe alkyloxy, un groupe alkylthio, un groupe alkyloxyalkyle, un groupe alkylthioalkyle, un groupe Ar-alkyle ou un groupe di(Ar)alkyle ;
p est un entier valant 1, 2, 3 ou 4 ;
R² est un atome d'hydrogène, un groupe hydroxy, un groupe mercapto, un groupe alkyloxy, un groupe alkyloxyalkyloxy, un groupe alkylthio, un groupe mono- ou di(alkyl)amino ou un radical de formule 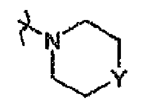 dans laquelle Y est un groupe CH₂, un atome d'oxygène, un atome de soufre, un groupe NH ou un groupe N-alkyle ;
R³ est un groupe alkyle, un groupe Ar, un groupe Ar-alkyle, un groupe Het ou un groupe Het-alkyle ;
q est un entier valant zéro, 1, 2, 3 ou 4 ;
R⁴ et R⁵ sont, chacun indépendamment, un atome d'hydrogène, un groupe alkyle ou un groupe benzyle ; ou
R⁴ et R⁵, conjointement et avec l'atome d'azote auquel ils sont fixés, peuvent former un radical choisi parmi le groupe constitué d'un groupe pyrrolidinyle, d'un groupe 2*H*-pyrrolyle, d'un groupe 2-pyrrolinyle, d'un groupe 3-pyrrolinyle, d'un groupe pyrrolyle, d'un groupe imidazolidinyle, d'un groupe pyrazolidinyle, d'un groupe 2-imidazolinyle, d'un groupe 2-pyrazolinyle, d'un groupe imidazolyle, d'un groupe pyrazolyle, d'un groupe triazolyle, d'un groupe pipéridinyle, d'un groupe pyridinyle, d'un groupe pipérazinyle, d'un groupe imidazolidinyle, d'un groupe pyridazinyle, d'un groupe pyrimidinyle, d'un groupe pyrazinyle, d'un groupe triazinyle, d'un groupe morpholinyle et d'un groupe thiomorpholinyle, éventuellement substitué par un groupe alkyle, un groupe halogéno, un groupe halogénoalkyle, un groupe hydroxy, un groupe alkyloxy, un groupe amino, un groupe mono- ou di-alkylamino, un groupe alkylthio, un groupe alkyloxyalkyle, un groupe alkylthioalkyle et un groupe pyrimidinyle ;
R⁶ est un atome d'hydrogène, un groupe halogéno, un groupe halogénoalkyle, un groupe hydroxy, un groupe Ar, un groupe alkyle, un groupe alkyloxy, un groupe alkylthio, un groupe alkyloxyalkyle, un groupe alkylthioalkyle, un groupe Ar-alkyle ou un groupe di(Ar)alkyle ; ou
deux radicaux R⁶ voisins peuvent être pris conjointement pour former un radical bivalent de formule -CH=CH-CH=CH- ;
r est un entier valant 1, 2, 3, 4 ou 5 ; et
R⁷ est un atome d'hydrogène, un groupe alkyle, un groupe Ar ou un groupe Het ;
R⁸ est un atome d'hydrogène ou un groupe alkyle ;
R⁹ est un groupe oxo ; ou
R⁸ et R⁹ forment conjointement le radical =N-CH=CH- ;
alkyle est un radical hydrocarboné saturé, linéaire ou ramifié, renfermant de 1 à 6 atomes de carbone ; ou est un radical hydrocarboné, saturé, cyclique, renfermant de 3 à 6 atomes de carbone ; ou est un radical hydrocarboné, saturé, cyclique, renfermant de 3 à 6 atomes de carbone, fixé à un radical hydrocarboné saturé, linéaire ou ramifié, renfermant de 1 à 6 atomes de carbone ; dans lequel chaque atome de carbone peut être éventuellement substitué par un groupe halogéno, un groupe hydroxy, un groupe alkyloxy ou un groupe oxo ;
Ar est un homocycle choisi parmi le groupe constitué d'un groupe phényle, d'un groupe naphtyle, d'un acénaphtyle, d'un groupe tétrahydronaphtyle, chacun éventuellement substitué par 1, 2 ou 3 substituants, chaque substituant étant choisi indépendamment parmi le groupe constitué d'un groupe hydroxy, d'un groupe halogéno, d'un groupe cyano, d'un groupe nitro, d'un groupe amino, d'un groupe mono- ou di-alkylamino, d'un groupe alkyle, d'un groupe halogénoalkyle, d'un groupe alkyloxy, d'un groupe halogénoalkyloxy, d'un groupe carboxyle, d'un groupe alkyloxycarbonyle, d'un groupe aminocarbonyle, d'un groupe morpholinyle et d'un groupe mono- ou dialkylaminocarbonyle ;
Het est un hétérocycle monocyclique choisi parmi le groupe constitué d'un groupe *N-*phénoxypipéridinyle, d'un groupe pyrrolyle, d'un groupe pyrazolyle, d'un groupe imidazolyle, d'un groupe furanyle, d'un groupe thiényle, d'un groupe oxazolyle, d'un groupe isoxazolyle, d'un groupe thiazolyle, d'un groupe isothiazolyle, d'un groupe pyridinyle, d'un groupe pyrimidinyle, d'un groupe pyrazinyle et d'un groupe pyridazinyle ; ou un hétérocycle bicyclique choisi parmi le groupe constitué d'un groupe quinoléinyle, d'un groupe quinoxalinyle, d'un groupe indolyle, d'un groupe benzimidazolyle, d'un groupe benzoxazolyle, d'un groupe benzisoxazolyle, d'un groupe benzothiazolyle, d'un groupe benzisothiazolyle, d'un groupe benzofuranyle, d'un groupe benzothiényle, d'un groupe 2,3-dihydrobenzo[1,4]dioxinyle ou d'un groupe benzo[1,3]dzoxolyle ; chaque hétérocycle monocyclique et bicyclique peut être éventuellement substitué sur un atome de carbone par 1, 2 ou 3 substituants choisis parmi le groupe constitué d'un groupe halogéno, d'un groupe hydroxy, d'un groupe alkyle ou d'un groupe alkyloxy ;
halogéno est un substituant choisi parmi le groupe constitué d'un groupe fluoro, d'un groupe chloro, d'un groupe bromo et d'un groupe iodo ; et
halogénoalkyle est un radical hydrocarboné saturé, linéaire ou ramifié, renfermant de 1 à 6 atomes de carbone ou un radical hydrocarboné saturé cyclique, renfermant de 3 à 6 atomes de carbone, dans lequel un ou plusieurs atomes de carbone sont substitués par un ou plusieurs atomes d'halogène.

2. Utilisation selon la revendication 1, dans laquelle R⁶, dans 1a formule (Ia) ou (Ib), est un atome d'hydrogène, un groupe halogéno, un groupe halogénoalkyle, un groupe hydroxy, un groupe Ar, un groupe alkyle, un groupe alkyloxy, un groupe alkylthio, un groupe alkyloxyalkyle, un groupe alkylthioalkyle, un groupe Ar-alkyle ou un groupe di(Ar)alkyle.

3. Utilisation selon 1a revendication 1 ou 2, dans laquelle, dans la formule (Ia) ou (Ib), R¹ est un groupe halogéno.

4. Utilisation selon la revendication 3, dans laquelle, dans la formule (Ia) ou (Ib), R¹ est un groupe bromo.

5. Utilisation selon l'une quelconque des revendications précédentes, dans laquelle, dans la formule (Ia) ou (Ib), p vaut 1.

6. Utilisation selon l'une quelconque des revendications précédentes, dans laquelle, dans la formule (la) ou (Ib), R² est un groupe alkyloxy.

7. Utilisation selon la revendication 6, dans laquelle, dans la formule (Ia) ou (Ib), R² est un groupe alkyloxy en C₁₋₄.

8. Utilisation selon la revendication 7, dans laquelle, dans la formule (Ia) ou (Ib), R² est un groupe méthyloxy.

9. Utilisation selon l'une quelconque des revendications précédentes, dans laquelle, dans la formule (Ia) ou (Ib), R³ est un groupe naphtyle ou un groupe phényle, chacun étant éventuellement substitué par un groupe halogéno.

10. Utilisation selon la revendication 9, dans laquelle R³ est un groupe naphtyle.

11. Utilisation selon l'une quelconque des revendications précédentes, dans laquelle, dans la formule (Ia) ou (Ib), q vaut 1.

12. Utilisation selon l'une quelconque des revendications précédentes, dans laquelle, dans la formule (la) ou (Ib), R⁴ et R⁵ sont chacun indépendamment un atome d'hydrogène ou un groupe alkyle.

13. Utilisation selon la revendication 12, dans laquelle R⁴ et R⁵ sont chacun indépendamment un groupe alkyle en C₁₋₄.

14. Utilisation selon la revendication 13, dans laquelle R⁴ et R⁵ sont chacun indépendamment un groupe méthyle.

15. Utilisation selon l'une quelconque des revendications précédentes, dans laquelle, dans la formule (Ia) ou (Ib), R⁶ est un atome d'hydrogène.

16. Utilisation selon l'une quelconque des revendications précédentes, dans laquelle, dans la formule (Ia) ou (Ib), R⁷ est un atome d'hydrogène.

17. Utilisation selon l'une quelconque des revendications précédentes, dans laquelle, dans la formule (Ia) ou (Ib), halogénoalkyle est un groupe trifluorométhyle.

18. Utilisation selon l'une quelconque des revendications précédentes, dans laquelle, dans la formule (Ia) ou (Ib), alkyle est un groupe méthyle ou un groupe éthyle.

19. Utilisation selon l'une quelconque des revendications précédentes, dans laquelle le dérivé de quinoléine substitué est un composé selon la formule (Ia).

20. Utilisation selon 1a revendication 1, dans laquelle le dérivé de quinoléine substitué est un composé selon la formule (Ia), dans laquelle R¹ est un atome d'hydrogène, un groupe halogéno, un groupe Ar, un groupe alkyle ou un groupe alkyloxy, p vaut 1, R² est un atome d'hydrogène, un groupe alkyloxy ou un groupe alkylthio, R³ est un groupe naphtyle, un groupe phényle ou un groupe thiényle, chacun étant éventuellement substitué par 1 ou 2 substituants choisis parmi le groupe constitué d'un groupe halogéno et d'un groupe halogénoalkyle, q vaut 0, 1, 2 ou 3, R⁴ et R⁵ sont chacun indépendamment un atome d'hydrogène ou un groupe alkyle, ou R⁴ et R⁵, conjointement et avec l'atome d'azote auquel ils sont fixés, forment un radical choisi parmi le groupe constitué d'un groupe imidazolyle, d'un groupe triazolyle, d'un groupe pipéridinyle, d'un groupe pipérazinyle et d'un groupe thiomorpholinyle, R⁶ est un atome d'hydrogène, un groupe alkyle ou un groupe halogéno, r vaut 0 ou 1 et R⁷ est un atome d'hydrogène.

21. Utilisation selon 1a revendication 1, **caractérisée en ce que** le composé est choisi parmi le groupe constitué :
o du 1-(6-bromo-2-méthoxy-quinoléin-3-yl)-2-(3,5-difluorophényl)-4-diméthylamino-1-phénylbutan-2-ol ;
o du 1-(6-bromo-2-méthoxy-quinoléin-3-yl)-4-diméthylamino-2-naphtalén-1-yl-1-phényl-butan-2-ol ;
o du 1-(6-bromo-2-méthoxy-quinoléin-3-yl)-2-(2,5-difluorophényl)-4-diméthylamino-1-phénylbutan-2-ol ;
o du 1-(6-bromo-2-méthoxy-quinoléin-3-yl)-2-(2,3-difluorophényl)-4-diméthylamino-1-phénylbutan-2-ol ;
o du 1-(6-bromo-2-méthoxy-quinoléin-3-yl)-4-diméthylamino-2-(2-fluorophényl)-1-phényl-butan-2-ol ;
o du 1-(6-bromo-2-méthoxy-quinoléin-3-yl)-4-diméthylamino-2-naphtalén-1-yl-1-p-tolyl-butan-2-ol ;
o du 1-(6-bromo-2-méthoxy-quinoléin-3-yl)-4-diméthylamino-2-naphtalén-1-yl-1-phénylbutan-2-ol ;
o du 1-(6-bromo-2-méthoxy-quinoléin-3-yl)-4-diméthylamino-2-(3-fluorophényl)-1-phényl-butan-2-ol ; et
o du 1-(6-bromo-2-méthoxy-quinoléin-3-yl)-4-diméthylamino-2-phényl-1-phénylbutan-2-ol ;
d'un sel pharmaceutiquement acceptable d'addition à un acide ou à une base de celui-ci, d'une forme stéréochimiquement isomère de celui-ci, ou d'une forme *N-*oxyde de celui-ci.

22. Utilisation selon la revendication 21, dans laquelle le composé est choisi parmi le groupe constitué :
o du 1-(6-bromo-2-méthoxy-quinoléin-3-yl)-4-diméthylamino-2-(3-fluorophényl)-1-phényl-butan-2-ol ;
o du 1-(6-bromo-2-méthoxy-quinoléin-3-yl)-4-diméthylamino-2-phényl-1-phénylbutan-2-ol ;
o du 1-(6-bromo-2-méthoxy-quinoléin-3-yl)-4-diméthylamino-2-naphtalén-1-yl-1-phényl-butan-2-ol ;
d'un sel pharmaceutiquement acceptable d'addition à un acide ou à une base de celui-ci, d'une forme stéréochimiquement isomère de celui-ci, ou d'une forme *N-*oxyde de celui-ci.

23. Utilisation selon la revendication 1, dans laquelle le composé est le 6-bromo-α-[2-(diméthylamino)éthyl]-2-méthoxy-α-1-naphtalényl-β-phényl-3-quinoléine-éthanol, un sel pharmaceutiquement acceptable d'addition à un acide ou à une base de celui-ci, une forme stéréochimiquement isomère de celui-ci, ou une forme *N-*oxyde de celui-ci.

24. Utilisation selon la revendication 23, dans laquelle le composé est le 6-bromo-α-[2-(diméthylamino)éthyl]-2-méthoxy-α-1-naphtalényl-β-phényl-3-quinoléine-éthanol ou un sel pharmaceutiquement acceptable d'addition à un acide de celui-ci.

25. Utilisation selon la revendication 23, dans laquelle le composé est le 6-bromo-α-[2-(diméthylamino)éthyl]-2-méthoxy-α-1-naphtalényl-β-phényl-3-quinoléine-éthanol ou une forme stéréochimiquement isomère de celui-ci.

26. Utilisation selon la revendication 23, dans laquelle le composé est le 6-bromo-α-[2-(diméthylamino)éthyl]-2-méthoxy-α-1-naphtalényl-β-phényl-3-quinoléine-éthanol ou une forme *N-*oxyde de celui-ci.

27. Utilisation selon la revendication 23, dans laquelle le composé est le (αS, βR)-6-bromo-α-[2-(diméthylamino)éthyl]-2-méthoxy-α-1-naphtalényl-β-phényl-3-quinoléine-éthanol ou un sel pharmaceutiquement acceptable d'addition à un acide de celui-ci.

28. Utilisation selon la revendication 27, dans laquelle le composé est le (αS, βR)-6-bromo-α-[2-(diméthylamino)éthyl]-2-méthoxy-α-1-naphtalényl-β-phényl-3-quinoléine-éthanol.

29. Utilisation d'un dérivé de quinoléine substitué pour la préparation d'un médicament destiné au traitement d'une infection par une souche de *Mycobacterium* résistant aux médicaments, dans laquelle le dérivé de quinoléine substitué présente la structure suivante : 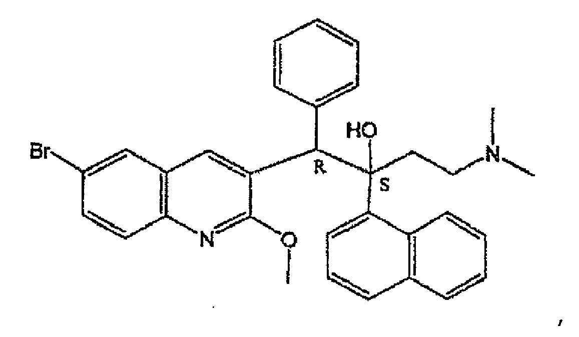 ou un sel pharmaceutiquement acceptable d'addition à un acide de celui-ci,

30. Utilisation selon l'une quelconque des revendications précédentes, dans laquelle la souche de *Mycobacterium* résistant aux médicaments, est résistante à des médicaments multiples.

31. Utilisation selon l'une quelconque des revendications précédentes, dans laquelle la souche de *Mycobacterium* est une souche de *Mycobacterium tuberculosis.*

32. Combinaison de (a) un composé de formule (la) ou (Ib), tel que défini dans l'une quelconque des revendications 1 à 29, et (b) un ou plusieurs autres agents anti-mycobactériens.

33. Combinaison de (a) un composé de formule (Ia) ou (Ib), tel que défini dans l'une quelconque des revendications 1 à 29, et (b) un ou plusieurs autres agents anti-mycobactériens, pour une utilisation en tant que médicament.

34. Composition pharmaceutique comprenant un support pharmaceutiquement acceptable et, en tant qu'ingrédient actif, une quantité thérapeutiquement efficace de (a) un composé de formule (Ia) ou (Ib), tel que défini dans l'une quelconque des revendications 1 à 29, et (b) un ou plusieurs autres agents anti-mycobactériens.

35. Produit contenant (a) un composé de formule (Ia) ou (Ib), tel que défini dans l'une quelconque des revendications 1 à 29, et (b) un ou plusieurs autres agents anti-mycobactériens, en tant que préparation combinée pour une utilisation simultanée, séparée ou séquentielle dans le traitement de maladies mycobactériennes.

36. Combinaison, composition pharmaceutique ou produit selon l'une quelconque des revendications 32 à 35, dans lesquels ledit ou lesdits autres agents anti-mycobactériens comprennent du pyrazinamide.

37. Combinaison, composition pharmaceutique ou produit selon l'une quelconque des revendications 32 à 35, dans lesquels ledit ou lesdits autres agents anti-mycobactériens sont choisis parmi la rifampicine (=rifampine) ; l'isoniazide ; le pyrazinamide ; l'amikacine ; l'éthionamide ; la moxifloxacine ; l'éthambutol ; la streptomycine ; l'acide paraaminosalicylique ; la cyclosérine ; la capréomycine ; la kanamycine ; la thioacétazone ; le PA-824 ; des quinolones/fluoroquinolones, telles que, par exemple, l'ofloxacine, la ciprofloxacine, la sparfloxacine ; des macrolides, tels que, par exemple, la clarithromycine, la clofazimine, l'amoxycilline avec l'acide clavulanique ; des rifamycines ; la rifabutine ; la rifapentine.

38. Combinaison, composition pharmaceutique ou produit selon l'une quelconque des revendications 32 à 37, dans lesquels le composé de formule (Ib) ou (Ib) est le (αS, βR)-6-bromo-α-[2-(diméthylamino)-éthyl]-2-méthoxy-α-1-naphtalényl-β-phényl-3 quinoléine-éthanol ou un sel pharmaceutiquement acceptable d'addition à un acide de celui-ci.

39. Combinaison, composition pharmaceutique ou produit selon l'une quelconque des revendications 32 à 38, dans lesquels le composé de formule (Ia) ou (Ib) est le (αS, βR)-6-bromo-α-[2-(diméthylamino)-éthyl]-2-méthoxy-α-1-naphtalényl-β-phényl-3-quinoléine-éthanol.

40. Utilisation d'une combinaison, d'une composition pharmaceutique ou d'un produit selon l'une quelconque des revendications 32 à 39, pour la préparation d'un médicament destiné au traitement d'une infection par une souche de *Mycobacterium* résistant aux médicaments.

41. Utilisation selon la revendication 40, dans laquelle la souche de *Mycobacterium* résistant aux médicaments est une souche de *Mycobacterium tuberculosis* résistant aux médicaments.
